(19) Europäisches Patentamt
European Patent Office
Office européen des brevets

(11) EP 1 478 652 B1

(12) # EUROPÄISCHE PATENTSCHRIFT

(45) Veröffentlichungstag und Bekanntmachung des Hinweises auf die Patenterteilung:
**25.04.2007 Patentblatt 2007/17**

(21) Anmeldenummer: **03742541.0**

(22) Anmeldetag: **19.02.2003**

(51) Int Cl.:
***C07F 9/6506*** *(2006.01)* ***C07K 5/062*** *(2006.01)*
***C07K 5/078*** *(2006.01)* ***C07K 5/09*** *(2006.01)*
***C07C 229/16*** *(2006.01)* ***C07D 233/64*** *(2006.01)*
***A61K 9/127*** *(2006.01)* ***C12N 15/88*** *(2006.01)*

(86) Internationale Anmeldenummer:
**PCT/EP2003/001662**

(87) Internationale Veröffentlichungsnummer:
**WO 2003/070735 (28.08.2003 Gazette 2003/35)**

(54) **KOMPONENTEN FUR DIE HERSTELLUNG AMPHOTERER LIPOSOMEN**
COMPONENTS FOR PRODUCING AMPHOTERIC LIPOSOMES
ELEMENTS PERMETTANT LA PREPARATION DE LIPOSOMES AMPHOTERES

(84) Benannte Vertragsstaaten:
**AT BE BG CH CY CZ DE DK EE ES FI FR GB GR HU IE IT LI LU MC NL PT SE SI SK TR**

(30) Priorität: **19.02.2002 DE 10207178**

(43) Veröffentlichungstag der Anmeldung:
**24.11.2004 Patentblatt 2004/48**

(73) Patentinhaber: **novosom AG**
**06120 Halle (DE)**

(72) Erfinder:
- **DR. ESSLER, Frank**
  **55131 Mainz (DE)**
- **Dr. PANZNER, Steffen**
  **06114 Halle (DE)**
- **ENDERT, Gerold**
  **06114 Halle (DE)**

(74) Vertreter: **Rasch, Dorit et al**
**Anwaltkanzlei**
**Gulde Hengelhaupt Ziebig & Schneider**
**Wallstrasse 58/59**
**10179 Berlin (DE)**

(56) Entgegenhaltungen:
**EP-A- 0 169 812** **WO-A-00/59474**
**WO-A-01/58849** **WO-A-95/35301**
**WO-A-02/072068** **US-A- 6 090 800**

- **HEYES J A ET AL: "Synthesis of novel cationic lipids: Effect of structural modification on the efficiency of gene transfer" JOURNAL OF MEDICINAL CHEMISTRY, AMERICAN CHEMICAL SOCIETY. WASHINGTON, US, Bd. 45, Nr. 1, 3. Januar 2002 (2002-01-03), Seiten 99-114, XP002219198 ISSN: 0022-2623**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft amphotere Verbindungen, auf Basis amphiphiler Moleküle, wobei an deren Kopfgruppen eine oder mehrere amphotere Gruppen mit einem isoelektrischen Punkt zwischen 4 und 9 substituiert sind, Liposomen, die diese Verbindungen enthalten sowie ihre Verwendung.

Unter dem Begriff der Lipide werden drei Klassen von Naturstoffen zusammengefasst, die sich aus biologischen Membranen isolieren lassen: Phospholipide, Sphingolipide und Cholesterol mit seinen Derivaten. Technisch hergestellte Verbindungen mit ähnlichen Eigenschaften sind die Diacylglycerole oder N, N-Dialkylamine.

Von technischem Interesse sind diese Substanzen bei der Herstellung von Liposomen. Diese Liposomen lassen sich unter anderem als Container für Wirkstoffe bei pharmazeutischen Zubereitungen einsetzen. Wünschenswert ist dabei eine effiziente und stabile Verpackung des Cargos und eine kontrollierbare Freisetzung des Inhalts. Beide Ansprüche sind nicht ohne weiteres zu vereinen: Je stabiler und dichter die Verpackung ist, desto schwerer gibt sie den eingeschlossenen Wirkstoff wieder frei. Aus diesem Grund wurden Liposomen entwickelt, die ihre Eigenschaften als Reaktion auf einen äußeren Reiz verändern. Bekannt sind thermosensible und pH-sensitive Liposomen. Die pH-sensitiven Liposomen sind von besonderem Interesse, da dieser Parameter sich auch unter physiologischen Umständen, etwa bei der endozytotischen Aufnahme eines Liposoms in Zellen oder bei der Passage des Magen-Darm-Trakts, ändern kann.

Nach dem Stand der Technik umfassen pH-sensitive Liposomen insbesondere Cholesterolhemisuccinat (CHEMS). Cholesterolhemisuccinat wird in Mischung mit Phosphatidylethanolamin zur Herstellung pH-sensitiver Liposomen verwendet (Tachibana et al.(1998); BBRC **251**: 538-544, US4891208). Solche Liposomen können von Zellen endozytiert werden und vermögen auf diesem Weg Cargomoleküle in das Innere von Zellen zu transportieren, ohne die Integrität der zellulären Membran zu verletzen.

Ein wesentlicher Nachteil des CHEMS ist dessen anionischer Charakter. Die damit hergestellten Liposomen besitzen eine negative Gesamtladung und werden nur mit geringer Effizienz von Zellen aufgenommen. Trotz des oben beschriebenen Transfermechanismus eignen sie sich daher kaum für den Eintransport von Makromolekülen in Zellen. Außerdem ist es nicht möglich makromolekulare Wirkstoffe wie DNA in diesen Liposomen zu verpacken.

Für den Eintransport von Wirkstoffen in Zellen (Transfektion) werden fachgemäß kationische Liposomen verwendet, die über eine möglichst hohe und konstante Oberflächenladung verfügen. Die positive Gesamtladung solcher Partikel führt zu einer elektrostatischen Anheftung an Zellen und in der Folge zu einem effizienten Eintransport. Der Einsatz dieser Verbindungen und der damit hergestellten Liposomen bleibt aber auf Anwendungen in vitro oder ex vivo beschränkt, da solche positiv geladenen Liposomen mit Serumbestandteilen unkontrollierte Aggregate bilden.

Der Stand der Technik beschreibt in der WO 00/59474 Verbindungen mit einem Membrananker und einer kationischen und anionischen Kopfgruppe auf demselben Molekül, wobei die anionische Gruppe über eine Disulfidbrücke mit der Grundstruktur verbunden ist. Diese Disulfidbrücke kann unter physiologischen Bedingungen, etwa bei Kontakt mit dem Cytosol, reduziert werden, die anionische Kopfgruppe wird dann freigesetzt und das Gesamtmolekül erhält eine positive Ladung und ermöglicht die Fusion mit der Zellmembran. Nachteilhaft ist das Toxizitätsprofil und die Lagerstabilität der in WO 00/59474 offenbarten Verbindungen, da durch die Abspaltung der Disulfidbrücken freie kationische Lipide entstehen. Für diese Verbindungen ist es bekannt, dass sie nachteilhafterweise eine zytotoxische Wirkung besitzen.

Weiterhin ist es bei den bekannten Lipiden und den sie umfassenden Liposomen nachteilig, dass diese nur unterdurchschnittliche Mengen an Proteinen, DNA und / oder RNA binden bzw. einschließen können. Sofern Veränderungen der Liposomen dahingehend vorgenommen werden, dass diese mehr Mengen an Carbo binden bzw. einschließen können, werden diese zytotoxisch oder weisen eine geringe Kompatibilität mit Serum oder Blut auf bzw. sind innerhalb des Blutes oder des Serums nicht stabil, da sie von einzelnen Bestandteilen des Blutes oder des Serums, wie beispielsweise Komplement oder Perforin, angegriffen werden. Die bekannten Liposomen und Lipide eignen sich daher nur bedingt für den Einsatz im lebenden Organismus und weisen weiterhin nur eine geringe Effizienz bei dem Transport von Inhalts- und / oder Bindungsstoffen auf. Einzelne, bekannte Verbindungen, die zumindest einen Teil der genannten Nachteile nicht aufweisen, sind nur sehr kompliziert herstellbar, wobei einzelne Komponenten so teuer sind, dass eine Verwertung im klinischen Maßstab bzw. bei der Verwendung in Laborroutinen nicht möglich ist.

Es bestand daher die Aufgabe, neue Verbindungen herzustellen, die die genannten Nachteile nicht aufweisen und

i) die es insbesondere ermöglichen, Wirkstoffe, insbesondere Plasmide in Liposomen einzuschließen,

ii) aus denen Liposomen hergestellt werden können, die einen eingeschlossenen Wirkstoff in das Innere von Zellen transportieren können,

iii) durch deren Anwesenheit die Herstellung von Liposomen möglich ist, die unter physiologischen Bedingungen

mit Serum gemischt werden können, ohne dass es zur Aggregation kommt,

iv) die in hohem Anteil in liposomale Membranen eingebaut werden können und

v) die sich einfach und preiswert herstellen lassen.

Die Erfindung löst diese technische Aufgabe durch Liposomen, umfassend amphiphile Verbindungen mit einem isoelektrischen Punkt zwischen 4,5 und 8,5, die ihren Ladungszustand reversibel von kationisch nach anionisch in einem pH-Bereich von 1-2 Einheiten ändern können, nach der allgemeinen Formel (I) :

(I) Amphoter - Y - **Spacer** - **Amphiphil**

wobei

(a) der **Amphoter** mindestens einen kationischen Ladungsteil mit einem pKa-Wert zwischen 4 bis 8 und/oder mindestens einen anionischen Ladungsteil mit einem pKa-Wert zwischen 3 bis 7 und gegebenenfalls weitere Ladungsträger umfasst, wobei

aa) der kationische Ladungsteil ausgewählt ist aus der Gruppe umfassend Imidazol, Morpholin, Piperazin, Purin, Pyridin und/oder Pyrimidin oder ein Derivat hiervon,

bb) der anionische Ladungsteil eine Carboxylgruppe ist, die in der aliphatischen Kette gebundene Essigsäure, Bromessigsäure, Chloressigsäure, Acetoessigsäure, Propionsäure, Acrylsäure, Buttersäure, Crotonsäure oder Carbonsäuren umfasst, die einfach veresterte oder amidierte oder in der aliphatischen Kette gebundene Dicarbonsäure wie Oxalsäure, Malonsäure, Bernsteinsäure, Maleinsäure, Fumarsäure, Äpfelsäure, Weinsäure, Glutarsäure, Adipinsäure, Caprylsäure, Pimelinsäure, Suberinsäure, Cyclohexandicarbonsäure oder auch Cyclopentandicarbonsäure umfasst; die einfach veresterte oder amidierte oder im aliphatischen Teil gebundene Oligocarbonsäure wie Citronensäure, Isocitronensäure oder Ethylendiamintetraessigsäure umfasst,

(b) der **Spacer** ein Niederalkylrest mit bis zu 8 C-Atomen mit linearer, verzweigter oder ringförmiger Struktur mit 0, 1 oder 2 ethylenisch ungesättigten Bindungen und 0-4 Hydroxylgruppen ist,

(c) **Y** -(C=O)-O-; -(C=O)-NH-; -NH-(C=O)-O-; -O-; -NH-; -CH=N-; -O-(O=C)-; -S-; (O=C)-; -NH-(O=C)-; -O-(O=C)-NH-, -N=CH- und/oder -S-S- umfasst.

(d) das Amphiphil eine Struktur nach der allgemeinen Formel (II) oder (III) oder (IV) ist:

(II)

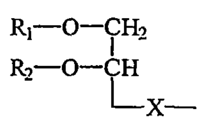

wobei
**R1** und **R2** unabhängig voneinander C8 bis C30 Alkyl oder Acylketten mit 0, 1 oder 2 ethylenisch ungesättigten Bindungen sind und
**X** ausgewählt ist aus der Gruppe umfassend -O-(C=O); -NH-(C=O)-; -S-(C=O)-; -O-; -NH-; -S-; -N=CH-; -(O=C)-O-; -S-(O=C)-; -NH-(O=C)-; -N=CH- und/oder -S-S-;
oder

(III)

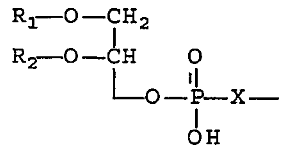

wobei
**R1** und **R2** unabhängig voneinander C8 bis C30 Acylketten mit 0, 1 oder 2 ethylenisch ungesättigten Bindungen sind und
**X** -O- ist.
oder

(IV)

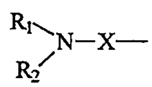

wobei
**R1** und **R2** unabhängig voneinander C8 bis C30 Alkylketten mit 0, 1 oder 2 ethylenisch ungesättigten Bindungen sind und
**X** abwesend oder ausgewählt ist aus der Gruppe bestehend aus -(C=O)-O-; -(C=O)-NH-; -(C=O)-S-; -NH-; -CH=N-; und/oder -S-(O=C)-;

(e) das Amphiphil eine mit linearen C8 bis C30-Alkoholen-veresterten 1,4- oder 1,5- Dicarbonsäuren wie. Asparaginsäure, Glutaminsäure, Äpfelsäure, Weinsäure, Citronensäure, Aconitsäure, Citraconsäure und/oder Maleinsäure ist und/oder

(f) das Amphiphil eine mit linearen C8 bis C30-Fettsäuren amidierten 1,4- oder 1,5- Diaminen des 3-Aminoalanins, Diaminobuttersäure, Ornithins oder Lysin ist.

Die Erfindung betrifft also die Lehre, dass durch Konjugation von amphoteren Gruppen über einen Spacer an ein Amphiphil, das sich in liposomale Membranen einbauen läßt, Strukturen bereitstellen lassen, die insbesondere zur Herstellung von Liposomen verwendet werden können, die geeignet sind, im lebenden Organismus eingesetzt zu werden und hohe Mengen an Proteinen, Peptiden, Kohlenhydrate, DNA und / oder RNA binden und / oder transportieren können. Überraschenderweise sind die neuen Verbindungen geeignet zur Herstellung von Vesikeln oder Liposomen, die im Blut oder Serum nicht aggregieren, die durch Complementkomponenten nicht angegriffen werden, die nicht zytotoxisch sind und über mehrere Stunden im Blut oder Serum stabil sind, wobei die Permeabilität der Liposomen vom pH-Wert und damit vom Ladungszustand der Verbindungen abhängig ist. D.h. die Wirkstofffreigabe dieser nicht-aggregierenden, stabilen, nicht-zytotoxischen Liposomen erfolgt in Abhängigkeit vom pH-Wert des Mediums.
Je nach verwendetem Amphoter und Amphiphil werden Verbindungen erhalten, die bei einem pH-Wert zwischen 4 und 9 ihre Ladung ändern und sich überraschenderweise zu einem hohen Anteil in liposomale Membranen einbauen lassen.
Im Zusammenhang mit der Erfindung sollen folgende Abkürzungen verwendet werden:

| | |
|---|---|
| CHEMS | Cholesterolhemisuccinat |
| PC | Phospatidylcholin |
| PE | Phosphatidylethanolamin |
| PS | Phosphatidylserin |

**Amphiphil**

Die in diesem Molekülbaustein vorhandenen ein oder zwei langkettigen Alkyle oder Acyle umfassen zwischen 8 und 30 C-Atome. Sie sind vorzugsweise geradkettig oder wenig verzweigt und können 0,1, oder 2 ethylenisch ungesättigte Bindungen aufweisen. Besonders bevorzugt sind solche Substituenten, die man in natürlichen Lipiden findet, also geradkettige Fettsäuren oder Alkohole mit 12 bis 20 C-Atomen und keiner, einer oder zwei ungesättigten Bindungen. Ganz besonders bevorzugt sind Lauroyl-, Myristoyl-, Palmitoyl-, Stearoyl-, Oleoyl- und Linoyl-Reste, beziehungsweise deren entsprechende Fettalkohole.

Bevorzugt werden als Amphiphile Diacylglycerole, Dialkylglycerole, Phosphoglycerole, acylierte oder alkylierte 3-Amino-1,2-Propandiole oder auch die N,N-Dialkylamine eingesetzt, da diese Verbindungen insbesondere billig verfügbar sind, eine einfache Chemie aufweisen und in einem hohen Anteil in Membranen eingebaut werden können, ohne deren Permeabilität zu erhöhen oder gar den Membrancharakter völlig zu zerstören.

In einer vorteilhaften Ausführung der Erfindung werden Dicarbonsäuren als polare Kopfgruppe des Amphiphils eingesetzt, die zweckmässigerweise eine Ankopplung der letztlich ladungstragenden Substituenten über weitere funktionelle Gruppen erlauben. Bevorzugt für die daraus abgeleiteten Amphiphile stehen: langkettige Ester von 1,4- oder 1,5- Dicarbonsäuren, wie etwa Asparaginsäure, Glutaminsäure, Äpfelsäure, Weinsäure, Citronensäure, Aconitsäure, Citraconsäure, Maleinsäure oder ähnliche solcher Verbindungen mit Fettalkoholen. Besonders bevorzugt sind die Lauryl-, Myristyl-, Palmityl-, Stearyl-, Oleyl- und Linol-Ester der genannten Dicarbonsäuren. Weitere Molekülbausteine (Spacer, Amphoter) werden über die verbleibende Aminogruppe, Hydroxylgruppe, Carboxylgruppe oder über die Doppelbindung gekoppelt.

Andere vorteilhafte Amphiphile erhält man aus Diaminen mit einer weiteren funktionellen Gruppe etwa als Diamid des 3-Aminoalanins, der Diaminobuttersäure, des Ornithins oder Lysins mit langkettigen Fettsäuren. Darunter sind die Lauroyl-, Myristoyl-, Palmitoyl-, Stearoyl-, Oleoyl- und Linoyl-Reste besonders bevorzugt.

Letztlich lassen sich die erfindungsgemässen Verbindungen auch als Derivate des Sphingosins oder der Ceramide herstellen. Sie sind auch als Derivate langkettiger Vinylether oder Plasmalogene darstellbar.

Die mit Vorteil als Ausgangsstoff verwendeten Amphiphile können an ihrer hydrophilen Kopfgruppe verschieden funktionalisiert sein um zweckmäßigerweise eine beständige, aber auch biologisch abbaubare Ankopplung erlauben oder optional die Funktion eines räumlichen Spacers zu erfüllen. Besonders geeignet für eine direkte Kopplung sind die vorhandene Hydroxylgruppe oder eine Aminogruppe. Weiterhin geeignet sind auch Carboxylgruppen.

**Amphoter:** Das Gesamtmolekül erhält seine pH-abhängige Ladungscharakteristik durch die gleichzeitige Anwesenheit von kationischen und anionischen Gruppen im Molekülteil "Amphoter". Ein Amphoter ist insbesondere dadurch gekennzeichnet, dass bei einem bestimmten pH-Wert die Summe seiner Ladungsbestandteile gerade Null ist. Dieser Punkt wird als isoelektrischer Punkt (iP) bezeichnet. Oberhalb des iP ist die Verbindung negativ geladen, unterhalb des iP ist sie als positives Kation aufzufassen; wobei der iP der erfindungsgemäßen Verbindungen zwischen 4,5 und 8,5 liegt.

Die Gesamtladung des Moleküls bei einem bestimmten pH-Wert des Mediums kann wie folgt berechnet werden:

$$z= \Sigma ni^{\cdot} * ((qi-1)+(10(pK-pH)/(1+10(pK-pH))))$$

qi absolute Ladung der ionischen Gruppe unterhalb ihres pK (Beispiel: Carboxyl = 0, einfache Stickstoffbase = 1, zweifach veresterte Phosphatgruppe = -1)

ni Anzahl dieser Gruppen im Molekül

Eine erfindungsgemäße Verbindung entsteht beispielsweise durch Kopplung der Aminogruppe des Histidins an Dipalmitoylglycerolhemisuccinat. Das Produkt ist bei einem neutralem pH-Wert von 7 negativ geladen, da die vorhandene Carboxylfunktion im Wesentlichen vollständig dissoziiert vorliegt und die Imidazolfunktion nur gering aufgeladen ist. Bei einem sauren pH-Wert von etwa 4 sind die Verhältnisse umgekehrt: die Carboxylfunktion ist nun weitgehend entladen, die Imidazolgruppe jedoch im Wesentlichen vollständig protoniert, die Gesamtladung des Moleküls ist daher positiv.

Werden Phospholipide als Amphiphile verwendet, so muß die dort vorhandene konstante negative Ladung des Phosphats durch eine zusätzliche positive Ladung kompensiert werden, damit eine erfindungsgemäße Verbindung entsteht. Eine Verbindung, die die erfinderische Lehre verdeutlichen soll, entsteht durch Kopplung von Histidin an Phosphatidylserin. Dabei ist es unerheblich, ob die Kopplung zwischen der Carboxygruppe des Histidins und der Aminogruppe des PS erfolgt oder ob die Aminogruppe des Histidins an die Carboxylfunktion des PS gekoppelt wird. In beiden Fällen entstehen Moleküle, in denen eine freie Aminogruppe die konstant negative Ladung der Phosphatgruppe neutralisiert. Die verbleibende Carboxylgruppe und die Imidazolfunktion reagieren wie oben, so dass es zur beabsichtigten Charakteristik vor erfindungsgemäßen Strukturen kommt.

In einer bevorzugten Ausführungsvariante der Erfindung weist das Molekül einen isoelektrischen Punkt zwischen 4 und 8, bevorzugt zwischen 5 und 7 auf.

In einer weiteren bevorzugten Ausführungsvariante der Erfindung umfasst das Amphoter ein oder mehrere Kationen mit einem pKa-Wert zwischen 4 und 8 und gleichzeitig ein oder mehrere Anionen mit einem pKa-Wert zwischen 3 und 7. Amphotere können insbesondere aus zwei Ladungsträgern zusammengesetzt sein, die beide im benannten pH-Bereich zwischen 4 und 9 ihre Ladung wechseln. Der gleichzeitig stattfindende Verlust an anionische Ladung und Gewinn an kationische Ladung führt zu einem Ladungswechsel des Gesamtmoleküls.

Dabei umfasst eine besonders bevorzugte Ausführungsvariante Amphotere, bei denen die pKa-Werte von Kation und Anion höchstens 2 pH-Einheiten auseinahderliegen. Besonders vorteilhaft wirkt sich das auf die Schärfe des iP aus. Je enger die beiden pKa-Werte beieinander liegen, desto enger ist der pH-Bereich, in dem die Molekülladung von kationisch zu anionisch wechselt. Eine vorteilhafte Auswahl von Art und Anzahl der Kationen und Anionen kann der Fachmann anhand der oben genannten Formel vornehmen.

Es kann zweckmäßig sein, funktionelle Gruppen oder Molekülfragmente als Ladungsträger zu verwenden, die im pH-Bereich zwischen 4 und 9 voll dissoziiert vorliegen. Dazu gehören insbesondere Phosphorsäuregruppen, Sulfonsäuregruppen oder andere starke Anionen. Bevorzugt sind weiterhin die meisten primären, sekundären oder tertiären Aminogruppen. Dazu gehören die quartären Ammonium-, Amidinium-, Pyridimnium- und die Guanidinogruppen. Die Phosphorsäuregruppe der Phospholipide ist ein besonders vorteilhaftes Beispiel für diesen Molekülbestandteil.

Diese, feststehenden Ladungen des Amphoters müssen erfindungsgemäß durch die beschriebenen veränderlichen Ladungen überkompensiert werden. Dies ist nur möglich, wenn die veränderlichen Ladungsträger in einem Überschuss eingesetzt werden. Wird beispielsweise eine tertiäres Amin als Kation verwendet, so werden mindestens 2 Carboxygruppen benötigt, um ein Amphoter im Sinne der Erfindung zu erhalten. Bei nur einer Carboxylgruppe kann nur die positive Ladung des Amins kompensiert werden, das Molekül niemals vollständig umladen. Vorteilhaft bei der Verwendung voll dissoziierter Gruppen ist deren starke Polarität.

Amphotere können besonders bevorzugt als komplette Struktureinheiten vorliegen. Das ist vorzugweise der Fall bei den o-, m- oder p-Aminobenzoesäuren, der Imidazolcarbonsäure, der Imidazoldiessigsäure oder auch der Nicotinsäure oder Picolinsäure.

Weitere vorteilhafte Verbindungen sind beispielsweise w-(1-Piperazino)alkylcarbonsäuren, Urocansäure, 4-(2-Aminoethyl)-imidazol-maleinsäuremonoamid, 4-(2-Hydroxyethyl)imidazolmaleinsäuremonoester, (2-Aminoethyl)morpholin-maleinsäuremonoamid oder analoge Verbindungen.

**pH-sensitive Kationen mit einem pKa zwischen 4 und 8:** Bevorzugt ist das Kation ein Imidazol, Piperazin, Morpholin, Purin oder Pyrimidin. Weitere vorteilhafte Kationen mit dieser Eigenschaft sind im wesentlichen Stickstoffbasen. Insbesondere wenn die Stickstoffbasen als Ringsysteme vorliegen, existieren vorteilhafterweise Stellungsisomere, bei denen der verbindende Spacer an verschiedenen Positionen des organischen Kations substituiert ist. Durch die Stellungsisomerie lassen sich zweckmäßigerweise die pKa-Werte der organischen Kationen beeinflussen. Die dem zugrunde liegenden Regeln sind dem Fachmann bekannt. Alternativ können diese Einflüsse aus Tabellenwerken abgeschätzt werden (Handbook of Chemistry and Physics, 73. Band, S. 8 - 37 ff.).

Vorteilhafte organische Kationen sind die insbesondere folgenden Stoffklassen:

> o-, m-, p-Aniline; 2-,3- oder 4-substituierte Anisidine, Toluidine oder Phenetidine; 2-, 3-, 5-,6-, 7-oder 8-substituierte Benzimidazole, 2-, 3, 4- oder 5-substituierte Imidazole, 1-, oder 5-substituierte Isochinoline, 2-,3- oder 4- substituierte Morpholine, 2-,3- oder 4- substituierte Picoline, 1-, 2-,oder 3- substitutierte Piperazine, 2-, 5- oder 6-modifizierte Pterine, 3-,4-, 5-, 6- oder 9-substituierte Purine, 2- oder 3- substituierte Pyrazine, 3- oder 4- substituierte Pyridazine, 2-, 3- oder 4- modifizierte Pyridine, 2- , 4-, 5- oder 6-subsituierte Pyrimidine, 1-,2-,3-, 4-,5-, 6- oder 8- substituierte Chinoline, 2-, 4- oder 5-substituierte Thiazole, 2-, 4- oder 6- substituierte Triazine, oder auch Abkömmlinge des Tyrosins.

Besonders bevorzugt sind die genannten Piperazine, Imidazole und Morpholine, Purine oder Pyrimidine. Ganz besonders bevorzugt sind solche Molekülfragmente, wie sie in biologischen Systemen vorkommen, also beispielsweise 4-Imidazole (Histamine, Histidin selbst), 2-,6- oder 9- Purine (Adenine, Guanine, Adenosine oder Guanosine), 1-, 2- oder 4-Pyrimidine (Uracile, Thymine, Cytosine, Uridine, Thymidine, Cytidine) oder auch Pyridin-3-carbonsäuren. Die hier genannten Strukturfragmente können weitere Substituenten aufweisen. Das können beispielsweise Methyl-, Ethyl-, Propyl- oder Isopropylreste sein, besonders bevorzugt in hydroxylierte Form mit einer oder zwei Hydroxylgruppen. Das können aber auch Hydroxyl- oder Ketofunktionen des Ringsystems sein.

Stickstoffbasen mit bevorzugten pKa-Werten entstehen beispielsweise auch durch einfache oder mehrfache Substitution eines Amins mit Niederalkanhydroxylen, etwa Hydroxymethyl- oder Hydroxyethylgruppen. Geeignete organische Basen aus dieser Gruppe sind beispielsweise Aminopropandiole, Triethanolamine, Tris-(hydroxymethyl)methylamine, Bis-(hydroxymethyl)methylamine, Tris-(hydroxyethyl)methylamine, Bis-(hydroxyethyl)methylamine oder die

entsprechend substituierten Ethylamine.

Stickstoffbasen mit bevorzugten pKa-Werten sind auch unter den Aminozuckern oder Aminozuckeralkoholen zu finden.

**pH-sensitive Anionen des Amphoters mit einem pKa zwischen 3 und 7:** Bevorzugt sind die anionischen Ladungsträger Carboxylgruppen. Selbstverständlich können alle Carbonsäuren als Ladungsträger eingesetzt werden. Dazu gehören insbesondere aliphatische, geradkettige oder verzweigte Carbonsäuren mit bis zu 8 C-Atomen und 0, 1 oder 2 ethylenisch ungesättigten Bindungen. Beispielhafte Verbindungsteile sind die Carboxylgruppe selbst, die in der aliphatischen Kette gebundene Essigsäure, Bromessigsäure, Chloressigsäure, Acetoessigsäure, Propionsäure, Acrylsäure, Buttersäure, Crotonsäure oder höhere Carbonsäure, die einfach veresterte oder amidierte oder in der aliphatischen Kette gebundene Dicarbonsäure wie Oxalsäure, Malonsäure, Bernsteinsäure, Maleinsäure, Fumarsäure, Äpfelsäure, Weinsäure, Glutarsäure, Adipinsäure, Caprylsäure, Pimelinsäure, Suberinsäure, Cyclohexandicarbonsäure oder auch Cyclopentandicarbonsäure; die einfach veresterte oder amidierte oder im aliphatischen Teil gebundene Oligocarbonsäure wie Citronensäure, Isocitronensäure oder Ethylendiamintetraessigsäure.

Weitere vorteilhafte Verbindungsteile sind die in der Seitenkette über ein Heteroatom gebundene Glykolsäure, Milchsäure, Hydroxybuttersäure, Äpfelsäure, Weinsäure, Asparaginsäure oder Glutaminsäure, Alanin, Glycin, Serin, Threonin, Asparagin, Glutamin, Prolin, Tyrosin oder Cystein oder andere Aminosäuren oder Hydroxysäuren.

Carbonsäuren mit einem geeigneten Verhalten findet man auch als Substituenten aromatischen Systeme, etwa als Benzoesäure, Anissäure, o-, m- oder p-Hydroxybenzoesäure, als Dihydroxybenzoesäure, Gallussäure, Zimtsäure, Phenylessigsäure, Hippursäure, Pthalsäure, Terephtalsäure, 2,3 oder 4-Pyridincarbonsäure, Furancarboxylsäure. Andere anionische Gruppen sind dissoziierbare Hydroxyle oder Thiole, wie sie in der Ascorbinsäure, dem N-substituierten Alloxan, der N-substituierten Barbitursäure, im Veronal, dem Phenol oder als Thiolgruppe vorkommen.

**Peptide als Amphotere:** In einer bevorzugten Ausführungsform der Erfindung sind die Amphotere Peptide und umfassen 2 bis 6 Aminosäuren. Besonders bevorzugt sind in einer weiteren Ausführungsform insbesondere die Aminosäuren Histidin, Arginin, Lysin, Glutaminsäure oder Asparaginsäure zur Bildung des Amphoters und zur Bestimmung seiner Ladungscharakteristik. Weitere bevorzugte Aminosäuren sind Glycin, Serin, Threonin, Glutamin, Asparagin, aber auch Cystein, die zur Erhöhung der Polarität und damit zur Verbesserung der Löslichkeit des Amphoters beitragen. Besonders bevorzugte Zusammensetzungen der Peptide zeigt die folgende Tabelle 1 in prozentualem Anteil an Gesamtaminosäure:

Tab.1

| *Aminosäure* | *His* | *Arg/Lys* | *Asp/Glu* | *Bedingungen* |
|---|---|---|---|---|
| i | Bis 66% | --- | Bis 66% | His, Asp/Glu ≠ 0 |
| ii | --- | < 50% | > Arg/Lys | Arg/Lys, Asp/Glu ≠ 0 |
| iii | ≤ 33% | ≤ 33% | > Arg/Lys | alle ≠ 0 |

Wobei i) den Fall zweier pH-sensitiver Komponenten, ii) den Fall einer feststehenden und einer pH-sensitiven Komponente und iii) den Fall einer Mischung von i) und ii) behandeln. Die Sequenz der einzelnen Aminosäuren ist beliebig, es kommt für die Ladungscharakteristik hauptsächlich auf die globale Zusammensetzung an. Terminale Gruppe des Peptids werden geblockt, im Falle des C-Terminus als Amid, im Fall des N-Terminus mit Acetyl.

**Spacer:** Zwischen dem Amphoter und dem Amphiphil liegen die Molekülfragmente: - Y - Spacer - X. Der Spacer ist ein Niederalkylrest mit linearer, verzweigter oder ringförmiger Struktur, der 0 bis 8 C-Atome besitzt und 0, 1 oder 2 ethylenische ungesättigte Bindungen enthält. Der Spacer kann zur Erhöhung der Polarität des Moleküls Hydroxylgruppen besitzen. Der Spacer kann insbesondere ein Zucker sein. Spacer kann vorteilhafterweise auch ein Polyethylenglykol sein, wobei dieser bis zu 20 Monomereinheiten umfassen kann.

**X und Y:** Bevorzugt umfasst die verbindende Gruppe X die Struktur -(C=O)-O-; -(C=O)-NH-; -NH-(C=O)-O-; -O-; -NH-; -CH=N- oder -S-S-. Vorteilhafterweise entspricht die verbindende Gruppe Y in ihrer Struktur der Gruppe X, zusätzlich kann sie die Struktur -O-(O=C)-; -S-; (O=C)-; -NH-(O=C)-; -O-(O=C)-NH- oder -N=CH- umfassen. X und / oder Y können auch Deletionen sein, d.h. ihre Anwesenheit ist nicht zwingend. Die Gruppe Y kann beispielsweise entfallen, wenn sich das Amphoter direkt an das Amphiphil koppeln lässt, beispielsweise bei der Veresterung von Imidazol-4,5-dicarbonsäure mit Dipalmitoylglycerol.

**Synthesemethoden:** Methoden zur Ausführung der chemischen Kopplung der einzelnen Molekülbausteine sind dem Fachmann bekannt und können je nach verwendetem Ausgangsstoff und Kopplungskomponente variieren. Typische Reaktionen sind die Veresterung, die Amidierung, die Addition von Aminen an Doppelbindungen, die Veretherung oder auch die reduktive Aminierung.

Besonders bevorzugte Moleküle lassen sich herstellen durch

i) Veresterung von Diacylglycerolen,

ii) Veresterung oder Amidierung von Diacylglycerolhemisuccinat,

iii) Addition von Aminen an die Doppelbindung eines Diacylglycerolhemimaleats,

iv) Amidierung von Phosphatidylethanolamin oder Phosphatidylserin,

v) Amidierung oder Alkylierung von 3-Amino-1,2-propandioldiestern,

vi) Oxidation von Phosphatidylglycerolen und anschliessende reduktive Aminierung und

vii) reduktive Aminierung von Glyceraldehyd und anschliessende Acylierung.

Zu den besonders bevorzugten Verbindungen gehören die folgenden Ausführungsformen (die langkettigen Kohlenwasserstoffketten der Amphiphile sind nur in verkürzter Schreibweise wiedergegeben und entsprechen Lauroyl-, Myristoyl-, Palmitoyl-, Stearoyl-, Oleoyl- und Linoyl-Resten):

Phospholipide

| | |
|---|---|
| 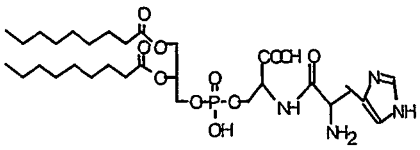 | **#7** Histidinyl-Phosphatidylserin durch Kopplung von Histidin an die Aminogruppe des Phosphatidylserin |
| 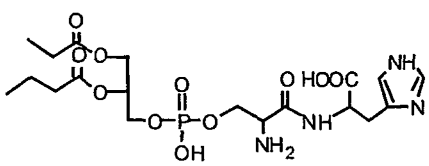 | **#26** Phosphatidylseryl-Histidin durch Kopplung von Histidin an die Carboxygruppe des Phosphatidylserin |
| 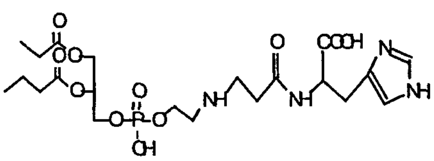 | **#25** Derivat des Phosphatidylglcerols. Die Ankopplung von Carnosin erfolgt nach Reaktion vi) |
| 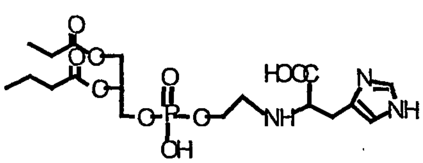 | **#29** Derivat des Phosphatidylglcerols . Die Ankopplung erfolgt nach Reaktion vi) mit Histidin |
| 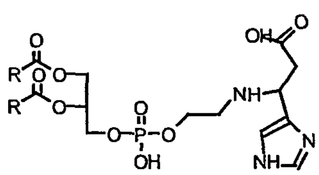 | **#28** N-Phosphatidylethyl-3-amino-5-imidazoylcarbonsäure: a) Addition des Phosphatidylethnanolamins an Urocansäure nach iii) oder b) nach vi) mit β-Histidin und Phosphatidylglycerol |

(fortgesetzt)

| | |
|---|---|
| 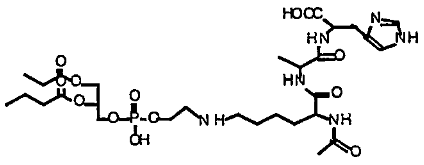 | **#31** Acetyl-Lys-Ala-His gekoppelt über die Seitenketten Aminogruppe des Lys an DPPG nach vi) |

1-Amino-2,3-Propandiole

| | |
|---|---|
| 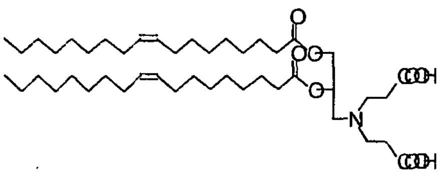 | **#8** Addition von Acrylsäure an 1-Amino-2,3-Propandiol und anschließende Acylierung |
| 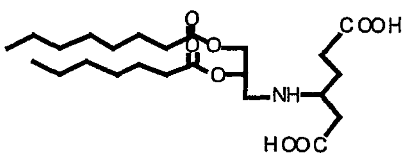 | **#32** Kondensationsprodukt aus Homoglutaminsäure und doppelt acyliertem 1-Iod-2,3-Propandiol |
| 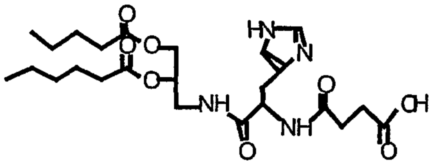 | **#36** Kopplung von CBZ-Histidin an doppelt acyliertes 1-Amino-2,3-Propandiol; Entschützen; Kopplung mit Succinanhydrid; |
| 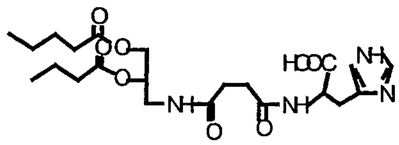 | **#37** Kopplung von Succinanhydrid andoppelt acyliertes 1-Amino-2,3-Propandiol; Kopplung von Benzyl-geschütztem Histidin; Entschützen |
| 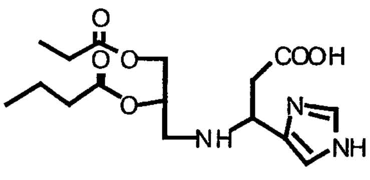 | **#38** Konjugation von β-Histidin an ein doppelt acyliertes 1-Jod-2,3-Propandiol |

Diacylglycerole

| | |
|---|---|
| 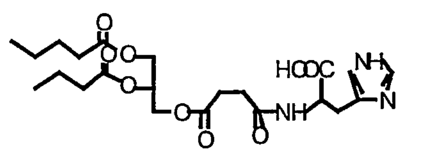 | **#34** Konjugation von Histidin an ein Diacylglycerolhemisuccinat |
| 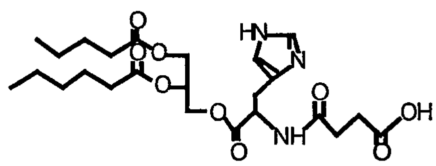 | **#35** Konjugation von Histidin an ein Diacylglycerol und anschließende Kopplung mit Succinanhydrid |

Dicarbonsäuren und Diamine

Die langkettigen Amphiphile sind nur in verkürzter Schreibweise wiedergegeben und entsprechen Lauryl-, Myristyl-, Palmityl-, Stearyl-, Oleyl- und Linyl-Resten.

| | |
|---|---|
| 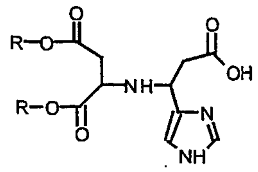 | **#50** N-(Asparaginsäuredihydroxyalkyl)-3-amino-5-imidazolcarbonsäure, kann durch Addition von Asparaginsäurediester an Urocansäure dargestellt werden. |
| 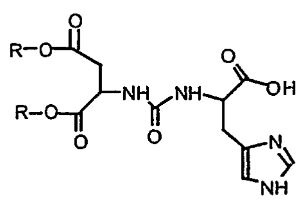 | **#51** N-(Aspartyldihydroxyalkyl)-N'-histidinyl-harnstoff. |
| 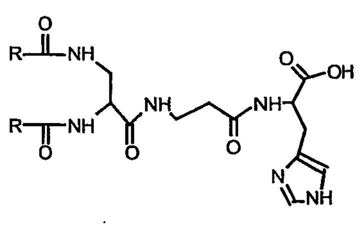 | **#52** doppelt acyliertes Tripeptid aus 2,3-Diaminopropionsäure, β-Alanin und Histidin. |

Zu den besonders bevorzugten amphoteren Komponenten gehören beispielsweise die folgenden Verbindungen, wobei R1 oder R2 das Amphiphil bedeuten und ( )n weitere Molekülteile im Sinne des oben definierten Spacers darstellen.

| | |
|---|---|
| 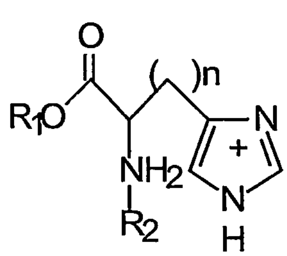 | Histidin-Derivate.<br>Bevorzugt erfolgt die Ankopplung des Amphiphils über die Aminogruppe als R2. R1 bildet in diesem Fall ein Anion und kann beispielsweise H oder eine Hydroxycarbonsäure oder eine oder mehrere Aminosäuren sein. Erfolgt die Ankopplung über R1, dann ist R2 ein anionischer Rest, etwa eine Carbonsäure oder Dicarbonsäure. |

(fortgesetzt)

| | |
|---|---|
| 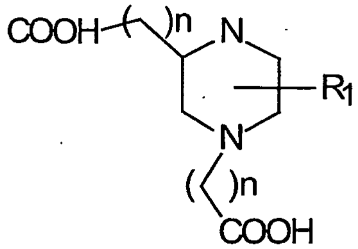 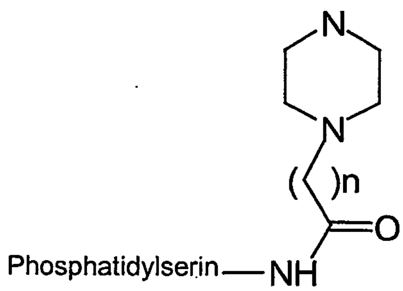 | Piperazin-Derivate.<br>Die Ankopplung des Amphiphils kann über eines der Ringatome erfolgen. Sind die Seitenketten als Hydroxycarbonsäuren oder Aminosäuren ausgeführt, so kann die Ankopplung vorteilhafterweise über diese Heteroatome erfolgen.<br>Das bevorzugte nebenstehende Derivat zeigt die Ankopplung von Piperazin an das Nα des Phosphatidylserin. |
| 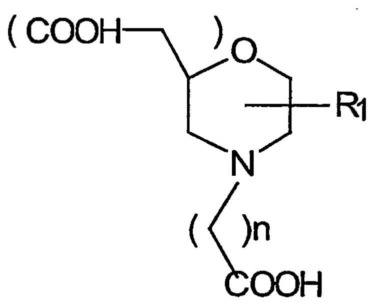 | Morpholin-Derivate<br>Die Ankopplung des Amphiphils kann über eines der Ringatome erfolgen. Sind die Seitenketten als Hydroxycarbonsäuren oder Aminosäuren ausgeführt, so kann die Ankopplung vorteilhafterweise über diese Heteroatome erfolgen. |
| 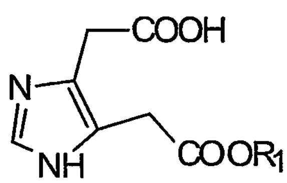 | Derivate der Imidazol-4,5-diessigsäure.<br>Die Anbindung des Amphiphils erfolgt bevorzugt als Ester einer der beiden Essigsäurefunktionen. Das Amphiphil kann aber auch an die 3-Aminofunktion angebunden sein. |
| 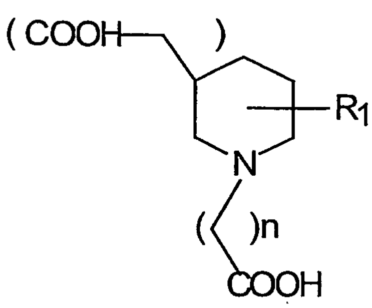 | Derivate des Piperidins.<br>Die Ankopplung des Amphiphils kann über eins der Ringatome erfolgen. Sind die Seitenketten als Hydroxy- oder Aminosäuren ausgeführt, dann kann die Ankopplung vertielhaft über deren Heteroatome erfolgen. |

(fortgesetzt)

| | |
|---|---|
| 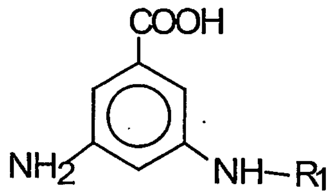 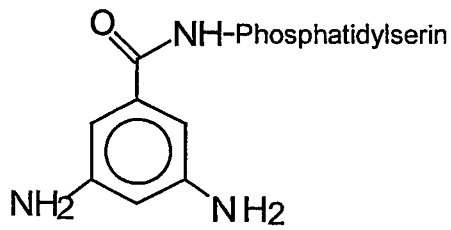 | Diaminobenzoesäure-Derivate.<br>Hier erfolgt die Ankopplung des Amphiphils bevorzugt über eine der beiden Aminogruppen. Die zweite Aminogruppe kann beispielsweise alkyliert sein, um einen höheren pKa-Wert zu erhalten.<br>Eine Ankopplung als Amid des Phosphatidylserins ist eine andere bevorzugte Ausführung der Erfindung. |
| 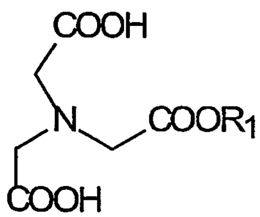 | Trinitriloessigsäure-Derivate.<br>Amphotere Grupen entstehen auch durch Veresterung von Trinitriloessigsäure. Das Ladungsverhalten dieser Verbindungen kann zusätzlich durch die Komplexierung von Metallionen verändert werden. |
| 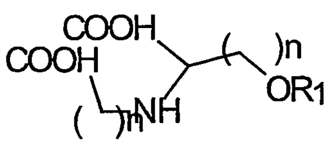 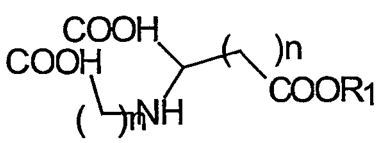 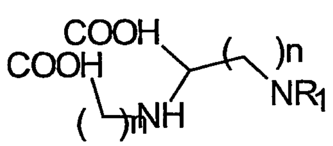 | Na-Alkylcarboxy-Aminosäure-Derivate<br>Amphotere Verbindungen entstehen auch hier durch Kopplung von Sterolen an die endständigen Gruppen von N-Acylaminosäuren. Die Struktur kann vorteilhaft vom Serin, der Asparaginsäure oder Glutaminsäure oder vom Lysin oder Ornithin abgeleitet werden. Die Aminodicarbonsäuren können nicht nur endständig, sondern auch an den anderen Säuregruppen gekoppelt sein. Das Ladungsverhalten dieser Verbindungen kann zusätzlich durch die Komplexierung von Metallionen verändert werden. |
| 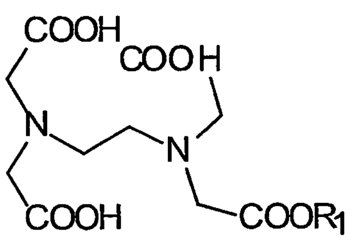 | EDTA-Derivate<br>Amphotere Grupen entstehen auch durch Veresterung von Trinitriloessigsäure. Das Ladungsverhalten dieser Verbindungen kann zusätzlich durch die Komplexierung von Metallionen verändert werden. |

Die Erfindung betrifft auch Liposomen, die die erfindungsgemäßen Verbindungen umfassen. Die erfindungsgemäßen Verbindungen lassen sich in einem hohen Anteil in liposomale Membranen einbauen und bilden amphotere Liposomen, die dadurch gekennzeichnet sind, dass sich ihr Ladungszustand reversibel durch pH-Änderung des umgebenden Mediums verändert. Unterhalb ihres isoelektrischen Punktes sind die Liposomen kationisch, oberhalb anionisch.

Liposomen, umfassend die erfindungsgemäßen Verbindungen können unter geeigneten Bedingungen mit

Polymeren beschichtet werden. Dabei kann eine einfache oder mehrfache Abscheidung solcher Substanzen auf der Oberfläche erfolgen. Bei einer mehrfachen Abscheidung, die gegebenenfalls unter Anwesenheit von Vernetzer durchgeführt wird, entstehen liposomale Nanokapseln. Verfahren zur Herstellung der Liposomen sind dem Fachmann z.B. aus der WO 00/28972 oder der WO 01/64330 bekann, die in dem Offenbarungsgehalt der Erfindung mit aufgenommen sind. Besonders vorteilhaft bei der Verwendung der erfindungsgemäßen Substanzen ist die Tatsache, dass eine elektrostatische Interaktion mit dem Polymer unterbrochen werden kann, wenn dieses ein Polyelektrolyt ist. Es ist bekannt, dass die Wechselwirkung eines Polyelektrolyten mit Ladungsträgern der liposomalen Membran zur Entmischung von Membranbestandteilen und zur Bildung von Lipidclustern führen kann. In vielen Fällen geht diese Entmischung mit einer Permeabilisierung des Liposoms einher. Die erfindungsgemäßen Substanzen ermöglichen eine Abschaltung dieser Wechselwirkung nach dem Beschichtungsprozess. Wird der pH-Wert zu diesem Zeitpunkt auf oder über den iP erhöht, so sind die Liposomen nur noch sterisch in der Nanokapseln eingeschlossen, eine Wechselwirkung der Membran mit den Polyelektrolyten besteht dann nicht mehr. Clusterbildung der Lipide und damit verbundene Permeabilisierung der Membran können so vorteilhafterweise umgangen werden.

Es wurde überraschend gefunden, dass Liposomen, deren Membran die erfindungsgemäßen Substanzen enthalten, unterhalb des isoelektrischen Punktes der Substanz leicht mit anderen Membranen, insbesondere Zellmembranen fusionieren. Für gewöhnlich erfordert dieser Schritt die Anwesenheit eines größeren Anteils von PE in der Membran. Dieses hat durch seine Neigung zur Bildung von hexagonalen Phasen die Funktion eines Helferlipids. Nachteilig ist aber die geringere Stabilität solcher Membranen, hier wird oft eine schleichende Freisetzung von eingeschlossenen Wirkstoffen beobachtet.

Liposomen, die unter Verwendung der erfindungsgemäßen Substanzen hergestellt werden, fusionieren vorteilhafterweise effektiv auch in Abwesenheit von Helferlipiden. Es sind also unter Verwendung der erfindungsgemäßen Substanzen Liposomen herstellbar, die einen Wirkstoff stabil verkapseln können, aber unter den Bedingungen eines niedrigen pH-Werts mit Zellmembranen fusionieren und dort den Wirkstoff freisetzen.

In einer bevorzugten Ausführungsform der Erfindung umfasst der Anteil der amphoteren Lipide maximal 30 mol%, 40 mol% oder 50 mol% des Gesamtlipids. Besonders vorteilhaft sind Zusammensetzungen, die mindestens 2 mol%, höchstens aber 50 mol% der amphoteren Lipide umfassen. Besonders bevorzugt sind Zusammensetzungen, die mindestens 5 mol%, bevorzugt 10 mol% und höchstens 50 mol%, bevorzugt 40 mol% des amphoteren Lipids umfassen. Die Herstellung von Liposomen umfassend die erfinderischen Substanzen erfolgt nach den dem Fachmann bekannten Techniken.

In einer weiteren bevorzugten Ausführungsvariante der Erfindung umfassen die Liposomen Phosphatidylcholin, Phosphatidylethanolamin, Diacylglycerole, Ceramide, Sphingolipide, Tetraetherlipide und/oder PEG-Lipide. Da die erfindungsgemäßen Verbindungen nicht immer selbst Liposomen bilden, kann es vorteilhaft sein, die genannten Lipide zuzusetzen.

In einer weiteren bevorzugten Ausführungsvariante der Erfindung weisen die Liposomen eine mittlere Größe zwischen 50 und 1000 nm, bevorzugt zwischen 50 und 500 nm, besonders bevorzugt zwischen 50 und 300 nm und ganz besonders bevorzugt zwischen 60 und 130 nm auf.

Zweckmäßig ist es, in die Liposomen insbesondere wasserlösliche Wirkstoffe einzuschließen. Sie können z.B. in der Krebstherapie und zur Therapie schwerer Infektionen verwendet werden. Liposomendispersionen können dazu injiziert, infundiert oder implantiert werden. Sie verteilen sich danach im Blut oder in der Lymphe oder geben als Depot ihren Wirkstoff kontrolliert ab. Letzteres kann durch hochkonzentrierte Dispersionen erreicht werden, die als Gele vorliegen. Die Liposomen können auch für topische Anwendung auf der Haut eingesetzt werden. Sie können insbesondere dazu beitragen, dass verschiedene Wirkstoffe besser in die Haut eindringen können oder sogar durch die Haut in den Körper gelangen können. Weiterhin ist es möglich die Liposomen für den Gentransfer einzusetzen. Genetisches Material kann wegen seiner Größe und Ladung meist nicht ohne Hilfsmittel in Zellen gelangen. Dazu bedarf es geeigneter Träger wie z.B. Liposomen oder Lipid-Komplexe. Diese sollen zusammen mit der DNA effizient und möglichst gezielt in die betroffenen Zellen aufgenommen werden. Besonders vorteilhaft ist es, wenn der Wirkstoff ein Protein, ein Peptid, eine DNA, eine RNA, ein antisense-Nukleotid und/oder ein Decoy-Nukleotid ist. Liposomen sind in ihrer prinzipiellen Struktur den Zellmembranen sehr ähnlich. Sie können daher als Membranmodelle eingesetzt werden, um die Permeationsgeschwindigkeit von Wirkstoffen durch Membranen oder die Membranbindung von Wirkstoffen zu quantifizieren.

In einer weiteren Ausführungsvariante der Erfindung sind mindestens 50μg, vorteilhafter 100μg, bevorzugt 150μg des Wirkstoffes pro mg Lipid in den Liposomen eingeschlossen. Nicht eingebaute, außen anhaftende Cargomoleküle können wenn nötig durch einen einfache Erhöhung des pH-Wertes entfernt werden. Dieser Schritt ist immer dann notwendig, wenn nicht eingebaute Cargomoleküle zu einer Aggregation der Liposomen führen. Vorteilhaft bei der Verwendung der erfindungsgemäßen Komponenten ist die Tatsache, dass die eingeschlossenen Wirkstoffe nur für den Zeitraum des eigentlichen Einschlusses unter Bedingungen gebracht werden müssen, die eine Interaktion mit der Lipidschicht erlauben. Sobald die Lipidschicht in sich geschlossen bleibt, kann zu anderen Bedingungen gewechselt werden. Eine denkbare Inaktivierung von Wirkstoffen, insbesondere von Proteinen kann dadurch minimiert werden.

Die Erfindung betrifft auch Verfahren zur Wirkstoffbeladung von Liposomen, wobei ein Bindungs-pH-Wert zur

Verkapselung benutzt wird und ein zweiter pH-Wert zur Abtrennung der nicht gebundenen Wirkstoffe verwendet wird.

Die Erfindung betrifft weiterhin ein Verfahren zur Wirkstoffbeladung von Liposomen, wobei die Liposomen bei einem bestimmten pH-Wert permeabilisiert und folgend verschlossen werden. Bevorzugt können insbesondere Permeabilitätsänderungen gezielt zur Beladung von Liposomen genutzt werden. Ein einzuschließender Wirkstoff kann dabei unter Bedingungen hoher Permeabilität ins Medium zugegeben werden. Anschließend werden Bedingungen geringer Permeabilität eingestellt. Damit verbleibt der Wirkstoff im Innern der Liposomen. Nicht eingeschlossener Wirkstoff kann dann gegebenenfalls abgetrennt werden. Eine solche Permeabilitätsänderung kann an Liposomen oder an liposomalen Nanokapseln herbeigeführt werden.

Die Erfindung betrifft auch die Verwendung der Liposomen in der Diagnostik und in Freisetzungssystemen. Die Liposomen können selbstverständlich auch in einem Detektionssystem verwendet werden. Insbesondere können die Liposomen mit Metallionen beladen werden, deren Fluoreszens durch die Chelatisierung verstärkt wird, also beispielsweise Terbium- oder Europiumionen. Liposomen für diese Anwendung können selbstverständlich spezifitätsbestimmende Komponenten enthalten, also z.B. Antikörper, Lektine, Selektine, Rezeptoren oder Hormone oder RNA-Aptamere. In einer besonders bevorzugten Ausführungsform der erfindungsgemäßen Verwendung ist die Anwesenheit dieser Metallionen auf das Lumen der Liposomen beschränkt, um unspezifische Signale von außen anhaftendem und langsam freigesetztem Metallionen zu vermeiden. Zweckmäßig ist es auch, die Liposomen zur Herstellung von Nanokapseln zu verwenden. Mit Vorteil können die Liposomen zur Herstellung von Freisetzungssystemen in der Diagnostik verwendet werden.

Zweckmäßig ist auch die Verwendung zum Transport und/oder zur Freisetzung von Wirkstoffen. Vorteilhafterweise können die Liposomen als Depotformulierung und/oder als zirkulierbares Depot verwendet werden. Vorteilhaft ist weiterhin die Verwendung der Liposomen als Vektor zur Transfektion von Zellen in vivo, in vitro und/oder ex vivo. Die Liposomen sind beispielsweise bei intravenöser und/oder peritonealer Applikation verwendbar.

Die erfindungsgemäßen Verbindungen und Liposomen weisen mehrere Vorteile auf. Überraschenderweise konnte festgestellt werden, dass die Permeabilität der erfindungsgemäßen Liposomen vom pH-Wert und damit vom Ladungszustand der Verbindungen abhängig ist.

Liposomen, die unter Verwendung der erfindungsgemäßen Strukturen hergestellt werden, sind daher insbesondere geeignet zur Konstruktion von Freisetzungssystemen, bei denen eine Abgabe von Wirkstoffen in Abhängigkeit vom pH-Wert des Mediums erfolgen soll.

Es wurde darüber hinaus überraschenderweise gefunden, dass in Liposomen, deren Membran die erfindungsgemäßen Verbindungen umfasst, überdurchschnittlich hohe Mengen, mindestens 50$\mu$g, bevorzugt 100$\mu$g, besonders bevorzugt 150$\mu$g an Proteinen oder DNA pro mg Lipid eingeschlossen werden können. Die Effizienz dieses Einbaus ist dabei abhängig vom pH-Wert der verwendeten Lösung. Ein Prozess für die effiziente Verkapselung von Proteinen oder DNA in die Liposomen kann daher so geführt werden, dass zunächst ein pH-Wert eingestellt wird, der zu einer guten Bindung der Cargomoleküle an die Lipidschicht führt. Für DNA als Polyanion werden hier niedrige pH-Werte von etwa 4 bis 5 verwendet. Bei Proteinen richtet sich der nutzbare pH-Wert nach dem isoelektrischen Punkt des Proteins. Dieser sollte unterhalb des isoelektrischen Punktes der erfindungsgemäßen Substanz liegen. Eine Verkapselung ist dann besonders effektiv, wenn der pH-Wert des Mediums so gewählt wird, dass er zwischen dem isoelektrischen Punkt des Proteins und dem isoelektrischen Punkt der erfindungsgemäßen Verbindung liegt. Das Protein ist dann negativ und die Lipidschicht ist positiv geladen.

Weiterhin wurde überraschend gefunden, dass Liposomen, deren Membran beispielsweise Histidinyl-PS oder Histidinyl-Diacylglycerolhemisuccinat umfasst, zur Chelatisierung von Metallionen befähigt sind. Diese Eigenschaft führt zu einer Verstärkung der positiven Ladung des Liposoms. Dieser Effekt ist besonders stark bei neutralen pH-Werten zu beobachten, da dann die Eigenladung der Verbindung gering ist. Aufgrund ihrer chelatisierenden Eigenschaften lassen sich solche Liposomen in der biochemischen Diagnostik und zur pharmazeutischen Therapie verwenden.

Eine wesentliche Voraussetzung für die Verwendung von Liposomen für experimentelle oder therapeutische Zwecke ist deren Verträglichkeit mit Zellen und Geweben. Eine Reihe bekannter Verbindungen, die für das Einbringen von DNA oder Proteinen in Zellen genutzt werden (beispielsweise das kationische Lipid DOTAP) sind zytotoxisch. Es wurde überraschenderweise gefunden, dass einige der erfindungsgemäßen Verbindungen eine verringerte Zytotoxizität zeigen. Das betrifft insbesondere die Gruppe von Verbindungen, bei denen das Amphoter eine Aminosäure oder ein Peptid ist. Diese erfüllen daher wesentliche Voraussetzungen eines Transfektionssystems.

Eine weitere Voraussetzung für die Konstruktion von Vektoren zum Gen- oder Proteintransport in Zellen ist deren Kompatibilität mit Serum oder Blut. Wegen ihrer starken kationischen Ladung bilden die derzeit bekannten Vektoren mit Serum unkontrollierte große Aggregate, die im Organismus zur Bildung von Thromben führen. Ihre Verwendung ist damit in vivo praktisch ausgeschlossen und auf in vitro oder ex vivo Anwendungen beschränkt. Es wurde überraschenderweise gefunden, dass Liposomen, die unter Verwendung der erfindungsgemäßen Komponenten aufgebaut wurden, in Serum oder Blut keine Aggregate bilden. Das sind insbesondere solche Liposomen, deren isoelektrischen Punkt kleiner als 7,5 ist.

Eine weitere Voraussetzung für die Konstruktion von Vektoren zum Protein- oder Gentransfer ist deren Stabilität

unter physiologischen Bedingungen. Liposomen werden bei einer Applikation in den Blutkreislauf vom Bestandteilen des Complementsystems angegriffen und schnell lysiert. Diese Reaktion erfolgt binnen Minuten. Es kommt zur Porenbildung in der Membran, durch die selbst grosse Moleküle wie Proteine ausdiffundieren können. Eine Stabilisierung von Liposomen gegenüber diesen Mechanismen ist bisher nur durch Einbau von Cholesteröl in die Lipidschicht möglich. Solche Liposomen sind dann sehr stabil, können aber nicht mehr mit Zellen wechselwirken oder ihren Wirkstoff leicht abgeben. Es wurde überraschenderweise gefunden, dass Liposomen, die unter Verwendung der erfindungsgemäßen Komponenten aufgebaut werden, in Serum oder Blut über mehrere Stunden stabil sein können. Die Wirkstofffreisetzung ist auch unter diesen Bedingungen gering. Eine liposomaler Vektor für den Transport von Wirkstoffen muss mindestens drei Voraussetzungen erfüllen: er muss eine geringe Toxizität besitzen, den Wirkstoff sicher und stabil einschließen und kompatibel mit Serum oder Blut sein.

Alle drei dieser Voraussetzungen werden von Liposomen, die unter Verwendung ausgewählter erfindungsgemäßer Substanzen hergestellt sind, mit Vorteil erfüllt. Die Liposomen sind daher für eine Verwendung bei therapeutischen Zwecken gut geeignet. Weitere Eigenschaften, die diese Verwendung unterstützen, sind die gute Beladbarkeit mit Wirkstoffen und die gezielte Ablösung dieser Stoffe durch Veränderungen des pH-Werts oder durch Permeabilisierung der Membran. Liposomen, die unter Verwendung der erfindungsgemäßen Substanzen hergestellt werden, zeigen nur eine geringe unspezifische Bindung an Zelloberflächen. Diese geringe unspezifische Bindung ist eine wesentliche Voraussetzung für das Zustandekommen einer spezifischen Bindung an Zielzellen. Werden die beschriebenen Liposomen mit weiteren Liganden versehen, so ist eine Zielsteuerung der Vehikel gegeben. Der Wirkstoff kann dann spezifisch an solchen Zellen oder Geweben angereichert werden, die pathologische Zustände aufweisen.

Eine wesentliche Verwendung der erfindungsgemäßen Substanzen liegt daher bei der Konstruktion von Vektoren für den Wirkstofftransfer in lebenden Organismen. Die Vektoren sind besonders geeignet für den Transport von therapeutischen Makromolekülen wie etwa Proteinen oder DNA, die von sich aus nicht die Zellmembran überwinden können oder im Blutstrom schnell abgebaut werden.

Überraschenderweise wurde gefunden, dass auch amphotere Amphiphile mit nur einer Kohlewasserstoffkette, wie sie in der US 6,255,344 offenbart sind, sich zur Herstellung von erfindungsgemäßen amphoteren Liposomen eignen, wenn die Kohlenwasserstoffkette mehr als 12 CH2-Gruppen umfasst. Diese Liposomen lassen sich mit Vorteil nach dem oben genannten Verfahren mit Wirkstoff, insbesondere DNA oder Oligonucleotiden beladen und ihre Verwendung zur Transfektion von Zellen sowie zur Gentherapie wird in den Offenbahrungsgehalt der vorliegenden Erfindung mit aufgenommen.

Ebenso wurde überraschenderweise gefunden, dass sich zweikettige Amphiphile, die durch Amidierung von langkettigen Fettsäuren mit langkettigen $\alpha$-amino-Carbonsäuren gebildet werden und mit einer amphoteren Gruppe, wie zum Beispiel Histidin versehen werden, sich ebenfalls zur Herstellung von amphoteren Liposomen eignen. Diese Liposomen lassen sich mit Vorteil nach dem oben genannten Verfahren mit Wirkstoff, insbesondere DNA oder oligonucleotiden beladen und ihre Verwendung zur Transfektion von Zellen sowie zur Gentherapie wird in den Offenbahrungsgehalt der vorliegenden Erfindung mit aufgenommen.

Die erfindungsgemäße Lehre soll an den folgenden Beispielen näher erläutert werden, ohne sich auf diese zu beschränken.

Beispiel 1

**Synthese von L-Histidinyl-Dipalmitoylglycerolsuccinat (DG-Succ-Hist, #34)**

Zur Synthese des DG-Succ-Hist werden 3 mmol (2 g) Diacylglycerol-Succinat (Avanti) mit 3,3 mmol (0,81 g) Benzyl-geschütztem Histidin, 3,45 mmol (0,71 g) DCC und 3,45 mmol (0,42 g) DMAP in 50 ml Dichlormethan als Lösungsmittel umgesetzt. Der Reaktionsansatz wird über Nacht bei Raumtemperatur Rühren gelassen. Nach erfolgter Amid-Kopplung wird die Carbonylgruppe des Histidins mittels katalytischer Hydrogenolyse an 10% Palladium/Kohlenstoff und Rühren über Nacht unter Wasserstoffatmosphäre entschützt. Nach Einengen des Reaktionsansatzes im Vakuum erfolgt die säulenchromatographische Reinigung an Kieselgel 60. Als Laufmittel wird Chloroform / Methanol / Ammoniak (25%ige Lösung) 60:40:2 verwandt.

Beispiel 2

**Synthese von Verbindung #35 (DG-Hist-Succ)**

Die Synthese des DG-Hist-Succ erfolgt in drei Stufen. Zunächst werden 3,7 mmol (2g) Dipalmitoylglycerol mit 4,1 mmol Aminogeschütztem CBZ-Histidin verestert. Die Esterbildung erfolgt unter Zugabe von 4,3 mmol (0,67 g) EDC und 4,3 mmol (0,53 g) DMAP in 60 ml Dichlormethan. Der Ansatz wird für 4 h gerührt. Durch Entschützung der Aminofunktion des Histidins entsteht das Zwischenprodukt DG-Hist. Die Aminogruppe wird mittels katalytischer Hydrogenolyse

an 10 % Palladium/Kohlenstoff durch Rühren über Nacht unter Wasserstoffatmosphäre entschützt. In einer zweiten Stufe wird Bernsteinsäureanhydrid mit Benzylalkohol zum Benzylsuccinat geöffnet. 10 mmol (1 g) Bernsteinsäureanhydrid werden mit 9,5 mmol (1 g) Benzylalkohol werden in 50 ml Toluol gelöst. Nach Zugabe von 1 mmol (190 mg) p-Toluolsulfonsäure-Monohydrat wird für zwei Stunden unter Rückfluß erhitzt. Im letzten Schritt werden 2 mmol (0,42 g) Benzyl-geschützte Succinat an 1,6 mmol (1,13g) DG-Hist über eine Amidbindung gekoppelt. Die Reaktion erfolgt unter Zugabe von 2,5 mmol (0,39 g) EDC und 2,5 mmol (0,3 g) DMAP in 50 ml Dichlormethan durch vierstündiges Rühren bei Raumtemperatur. Abschließend erfolgt die Entschützung des Succinats durch Abspaltung des Benzylrestes durch katalytische Hydrogenolyse an 10 % Palladium/Kohlenstoff durch Rühren über Nacht unter Wasserstoffatmosphäre. Nach Einengen des Reaktionsansatzes im Vakuum erfolgt die säulenchromatographische Reinigung an Kieselgel 60. Als Laufmittel wird Chloroform / Methanol /Ammoniak (25%ige Lösung) 60:40:2 verwandt.

Beispiel 3

**Synthese von Verbindung #8**

**(N,N-Bis(propansäure-3-yl)-N-(2,3-dioleoyloxy-propyl)amin**

Die Synthese der gewünschten Verbindung verläuft über drei Stufen.
In der 1. Stufe wird die Aminogruppe des 3-Amino-1,2-propandiols mit tert-Butylacrylat geschützt. 111 mmol (10 g) 3-Amino-1,2-propandiol werden in 100 ml Acetonitril vorgelegt. Unter Schutzgas werden 222 mmol (28,5 g) tert.-Butylacrylat zugegeben und der Ansatz für zwei Tage unter Rückfluß erhitzt. Die Reaktionsmischung wird am Rotationsverdampfer eingeengt und an Kieselgel säulenchromatographisch gereinigt. Als Laufmittel wurde Essigester / Methanol 9:1 verwandt. Im nächsten Schritt werden 17,7 mmol (6,15 g) Zwischenprodukt Stufe 1 mit 35,4 mmol (10 g) Ölsäure in 100 ml Dichlormethan vorgelegt. Die Reaktion wird unter Schutzgas und Eisbad-Kühlung durchgeführt. Nach Zugabe von 35,4 mmol (7,3 g) Dicyclohexylcarbodiimid wird die Eiskühlung entfernt und der Ansatz einen Tag bei Raumtemperatur gerührt. Der ausgefallene Harnstoff wird abfiltriert und die Lösung am Rotationsverdampfer eingeengt. Das Produkt wird roh weiterverwandt. Abschließend wird die Schutzgruppe abgespalten. Dazu werden 11,5 mmol Zwischenprodukt Stufe 2 in 25 ml Dichlormethan vorgelegt. Unter Eiswasserkühlung werden langsam 25 ml Trifluoressigsäure zugegeben. Es wird für einen Tag bei 40 ˚C gerührt. Nach Abziehen des Lösungsmittels wird der Rückstand säulenchromatographisch an Kieselgel gereinigt.
Als Lösungsmittel wird Essigester / Petrolether 1:1 verwandt. Das gewonnene Öl wird in circa 50 ml Aceton aufgenommen und mit 1 N Natriumhydrogencarbonatlösung leicht basisch eingestellt. Nach Zentrifugation wird die überstehende Lösung verworfen. Der Rückstand wird mit wenig Aceton im Utraschallbad behandelt, zentrifugiert und die überstehende Lösung wird verworfen. Der Rückstand wird in 30 ml Chloroform aufgenommen und mit 30 ml Wasser überschichtet. Unter kräftigem Rühren wird mit 1 N HCl auf pH 5 eingestellt. Die organische Phase wird abgetrennt, über Natriumsulfat getrocknet und am Rotationsverdampfer eingeengt.

Beispiel 4

**Herstellung von amphoteren Liposomen**

2 mg des entsprechenden Lipids und 10 mg DMPC werden in 4 mL Chloroform/ Methanol (1:1, v/v) gelöst und im Rotationsverdampfer vollständig getrocknet. Der Lipidfilm wird mit 4.3 mL eines Puffers (10 mM Kac, 10 mM HEPES, 150 mM NaCl, pH 7,5) in einer Lipidkonzentration von 5 mM durch 5 min Ultraschallbehandlung hydratisiert. Abschließend wird die Suspension eingefroren und nach dem Auftauen mehrfach extrudiert (Avestin LiposoFast, Polycarbonatfilter 200nm Porenweite). Der Verlauf des Zetapotentials in mV bei verschiedenen pH-Werten ist in Abbildung 1 dargestellt. Der Nulldurchgang zeigt den pH-wert, bei dem das Liposom entladen ist. Dieser pH entspricht dem isoelektrischen Punkt des Lipids und ist für #8 4,5; #34 5,2; #25 5,7;

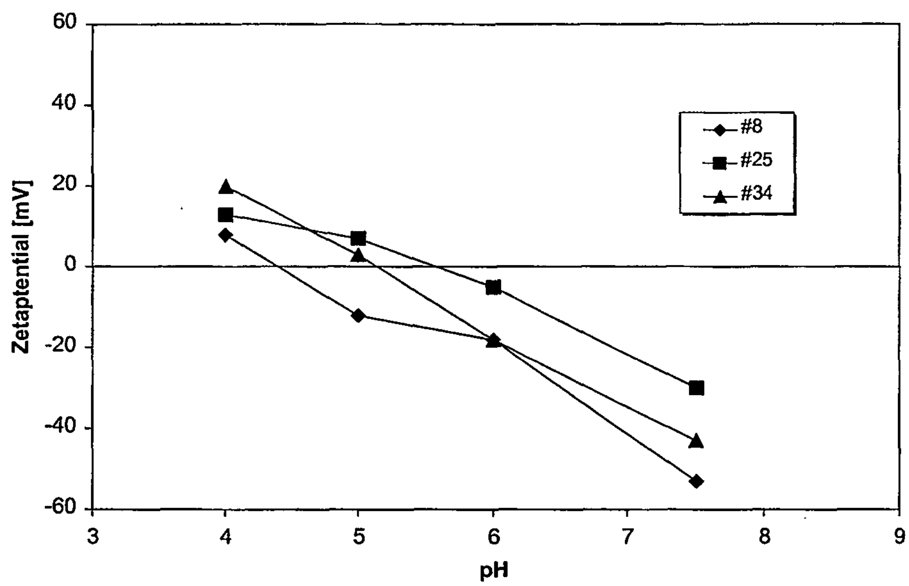


Abbildung 1

Beispiel 5

**In situ- Synthese von Verbindung #25 (Phosphatidylglyceryl-Carnosin)**

Unilamellare Liposomen (DPPC / DPPG / Cholesterol 40:20:40 Mol%) werden in einer Konzentration von 20mM Lipid in einem Borat-Puffern (20mM Natriumborat, 120 mM Natriumchlorid pH8,4) suspendiert. Zu 2ml dieser Lösung werden 400µl einer 0,6M Natriumperiodatlösung zugegeben, die Mischung wird für 30min im Dunkeln inkubiert. 1ml einer solchen Suspension wird an Sephadex G25 im oben verwendeten Boratpuffer chromatografiert. Das Eluat der Liposomensuspension wird auf 4ml aufgefüllt.
Zu den so oxidierten Liposomen wird Carnosin in einer Endkonzentration von 20mM zugegeben und 2 Stunden inkubiert. Abschliessend wurde mit 20mM Natriumborhydrid über Nacht bei 4°C reduziert. überschüssiges Carnosin kann durch Chromatografie an Sephadex G25 wie oben abgetrennt werden.

Beispiel 6

**Messung der Serumaggregation von Liposomen**

Zu 140µL Humanserum werden 10µl einer 25mM Liposomensuspension pipettiert und gut gemischt. Davon werden 65 µL abgenommen und mit 1,5 ml Puffer (HEPES 10mM, NaCl 150mM pH 7,5) verdünnt. Der Rest wird für 2 h bei 37°C inkubiert und wieder werden 65 µL abgenommen und mit 1,5 mL Puffer verdünnt. Von beiden Proben werden die Partikelgrößen mit einem Malvern Zetasizer 3000 bestimmt. Parallel dazu werden Kontrollproben nur in Puffer aufgenommen, die ebenfalls 2 h bei 37°C inkubiert wurden. Eine unveränderte Partikelgröße zeigt eine eine gute Serumverträglichkeit an.

Beispiel 7

**Herstellung von mit DNA-Plasmiden beladenen amphoteren Liposomen**

1,43 mM des amphoteren Lipids werden je nach Lipidzusammensetzung des Lipidfilmes mit den anderen Lipiden in Chloroform gelöst. Nach Abziehen des Lösungsmittels wird der Lipidfilm über Nacht im Vakuum getrocknet. Der Lipidfilm wird direkt mit 1 ml DNA-haltigen (100 µg DNA /ml) NaAc-Puffer (10 mM NaAc, 150 mM NaCl, pH 4) hydratisiert (leichter Ultraschall, anschließend für 30 min oberhalb der Phasenumwandlungstemperatur rotiert). Anschließend erfolgt ein freeze/thaw-Schritt.
Die Mischung wird 10 °C oberhalb der Phasenumwandlungstemperatur 15fach durch 400 nm-Membranen extrudiert.

Nicht eingeschlossenen DNA kann durch Flotation im Sucrose-Gradienten (bei pH 7.5) abgetrennt werden (0,8 M Sucrose, 0,5 M Sucrose, Puffer).
Der Gehalt an DNA wird mit Hilfe des Interkalationsfarbstoffes Propidiumiodid durch die Zunahme der Fluoreszenzintensität bei Interkalation in die DNA bestimmt. Dazu werden 20 µl Propidiumiodid, 6 µl Triton X-100 (10 % in Wasser) mit Probe auf 300µl aufgefüllt und mit einem Fluoreszenzplattenreader vermessen.

## Patentansprüche

1. Amphoteres Lipid mit einem isoelektrischen Punkt zwischen 4,5 und 8,5 nach der allgemeinen Formel (I):

(I) Amphoter - Y - Spacer - Amphiphil

**dadurch gekennzeichnet, dass**

(a) der **Amphoter** mindestens einen kationischen Ladungsteil mit einem pKa-Wert zwischen 4 bis 8 und/oder mindestens einen anionischen Ladungsteil mit einem pKa-Wert zwischen 3 bis 7 und gegebenenfalls weitere Ladungsträger umfasst, wobei

aa) der kationische Ladungsteil ausgewählt ist aus der Gruppe umfassend Imidazol, Morpholin, Piperazin, Purin, Pyridin und/oder Pyrimidin oder ein Derivat hiervon,
bb) der anionische Ladungsteil eine Carboxylgruppe ist, die in der aliphatischen Kette gebundene Essigsäure, Bromessigsäure, Chloressigsäure, Acetoessigsäure, Propionsäure, Acrylsäure, Buttersäure, Crotonsäure oder Carbonsäuren umfasst, die einfach veresterte oder amidierte oder in der aliphatischen Kette gebundene Dicarbonsäure umfasst; die einfach veresterte oder amidierte oder im aliphatischen Teil gebundene Oligocarbonsäure umfasst,

(b) der **Spacer** ein Niederalkylrest mit 0 bis 8 C-Atomen mit linearer, verzweigter oder ringförmiger Struktur mit 0, 1 oder 2 ethylenisch ungesättigten Bindungen und 0-4 Hydroxylgruppen ist,
(c) Y abwesend ist oder -(C=O)-O-; -(C=O)-NH-; -NH-(C=O)-O-; -O-; -NH-; -CH=N-; -O-(O=C)-; -S-;. (O=C)-; -NH-(O=C)-; -O-(O=C)-NH-, -N=CH- und/oder -S-S- umfasst
(d) das Amphiphil eine Struktur nach der allgemeinen Formel (II) oder (IV) ist:

(II)

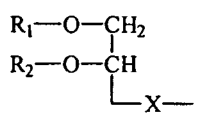

wobei
**R1** und **R2** unabhängig voneinander C8 bis C30 Alkyl oder Acylketten mit 0, 1 oder 2 ethylenisch ungesättigten Bindungen sind und
**X abwesend oder** ausgewählt ist aus der Gruppe umfassend -O-(C=O); -NH-(C=O)-; -S-(C=O)-; -O-; -NH-; -S-; -N=CH-; -(O=C)-O-; -S-(O=C)-; -NH-(O=C)-; -N=CH- und/oder -S-S-;
oder

(IV)

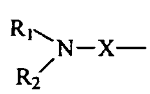

wobei
**R1** und **R2** unabhängig voneinander C8 bis C30 Alkylketten mit 0, 1 oder 2 ethylenisch ungesättigten Bindungen

sind und
**X** abwesend oder ausgewählt ist aus der Gruppe bestehend aus -(C=O)-O-; -(C=O)-NH-; -(C=O)-S-; -NH-; -CH=N-; und/oder -S-(O=C)-;
(e) das Amphiphil eine mit linearen C8 bis C30-Alkoholen-veresterten 1,4- oder 1,5-Dicarbonsäuren wie Asparaginsäure, Glutaminsäure, Äpfelsäure, Weinsäure, Citronensäure, Aconitsäure, Citraconsäure und/oder Maleinsäure ist und/oder
(f) das Amphiphil eine mit linearen C8 bis C30-Fettsäuren amidierten 1,4- oder 1,5- Diaminen des 3-Aminoalanins, Diaminobuttersäure, Ornithins oder Lysin ist.

**2.** Lipid nach Anspruch 1, **dadurch gekennzeichnet,**
**dass** die Dicarbonsäure ausgewählt ist aus der Gruppe umfassend Oxalsäure, Malonsäure, Bernseinsäure, Maleinsäure, Fumarsäure, Äpfelsäure, Weinsäure, Glutarsäure, Adipinsäure, Caprylsäure, Pimelinsäure, Suberinsäure, Cyclohexandicarbonsäure und / oder Cyclopentandicarbonsäure und / oder die Oligocarbonsäure ausgewählt ist aus der Gruppe umfassend Citronensäure, Isocitronensäure und / oder Ethylendiamintetraessigsäure.

**3.** Lipid nach Anspruch 1 oder 2,
**dadurch gekennzeichnet, dass**
das amphotere Lipid einen isoelektrischen Punkt zwischen 5 und 7 aufweist.

**4.** Lipid nach einem der Ansprüche 1 bis 3,
**dadurch gekennzeichnet, dass**
sich die pKa-Werte des kationischen und anionischen Ladungsteils des Amphoters sich höchstens um 2 pH-Einheiten unterscheiden.

**5.** Lipid nach einem der Ansprüche 1 bis 4,
**dadurch gekennzeichnet, dass**
das kationische Ladungsteil des Amphoters Imidazol, Piperazin, Morpholin, der anionische Ladungsteil die Carboxylgruppe umfasst.

**6.** Lipid nach einem der vorhergehenden Ansprüche 1 bis 5,
**dadurch gekennzeichnet, dass**
das Amphoter ein Peptid mit 2-6 Aminosäuren ist, die ausgewählt sind aus der Gruppe bestehend aus Histidin, Arginin, Lysin, Glutaminsäure und/oder Asparaginsäure,
wobei

i) der prozentuale Anteil von His und Asp/Glu 66% nicht überschreitet oder
ii) der prozentuale Anteil von Arg/Lys kleiner 50%, der von Asp/Glu größer als 50% ist, oder
iii) der prozentuale Anteil von His und Arg/Lys kleiner/gleich 33%, der von der von Asp/Glu größer als Arg/Lys ist.

**7.** Lipid nach einem der vorhergehenden Ansprüche 1 bis 6,
**dadurch gekennzeichnet, dass**
das **Amphiphil** ausgewählt ist aus der Gruppe bestehend aus Diacylglycerolen, Dialkylglycerolen, Phosphoglycerolen, acylierte oder alkylierte 3-Amino-1,2-Propandiolen und/oder N,N-Dialkylaminen.

**8.** Lipid nach einem der vorhergehenden Ansprüche 1 bis 7,
**dadurch gekennzeichnet, dass**
der Spacer Zucker oder ein Polyethylenglykol mit bis zu 20 Monomereinheiten umfasst.

**9.** Amphoteres Lipid nach einem der vorhergehenden Ansprüche mit der Struktur

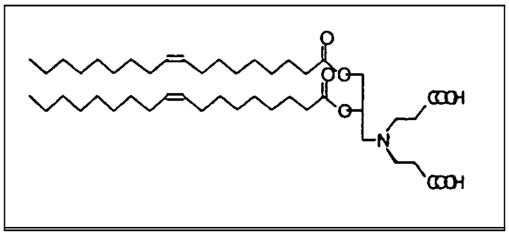

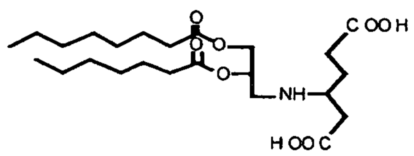

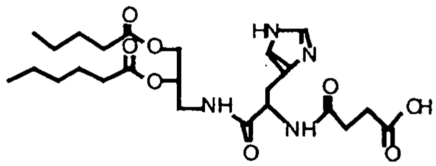

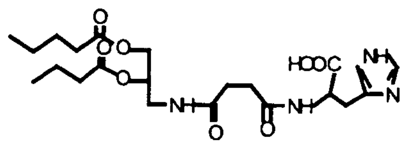

oder

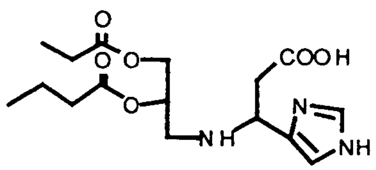

wobei die langkettigen Alkyle oder Acyle unabhängig voneinander

a) Lauroyl-, Myristoyl-, Palmitoyl-, Stearoyl-, Oleoyl- oder Linoyl-Reste oder
b) Lauryl-, Myristyl-, Palmityl-, Stearyl-, Oleyl- oder Linyl-Reste umfassen.

**10.** Amphoteres Lipid nach einem der vorhergehenden Ansprüche mit der Struktur

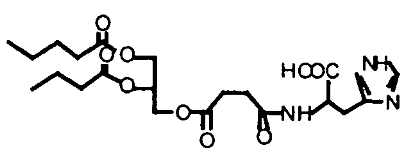

oder

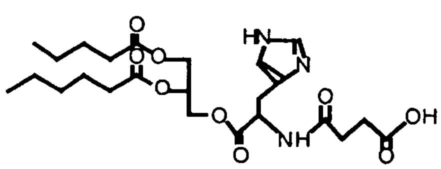

wobei die langkettigen Alkyle oder Acyle unabhängig voneinander

a) Lauroyl-, Myristoyl-, Palmitoyl-, Stearoyl-, Oleoyl- oder Linoyl-Reste oder
b) Lauryl-, Myristyl-, Palmityl-, Stearyl-, Oleyl- oder Linyl-Reste umfassen.

**11.** Amphoteres Lipid nach einem der vorhergehenden Ansprüche mit der Struktur

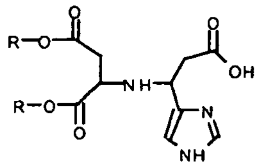

oder

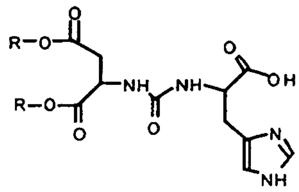

wobei die langkettigen Alkyle unabhängig voneinander Lauryl-, Myristyl-, Palmityl-, Stearyl-, Oleyl- oder Linyl-Reste umfassen.

**12.** Amphoteres Lipid nach einem der vorhergehenden Ansprüche mit der Struktur

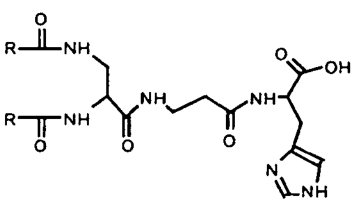

wobei die langkettigen Acyle Lauroyl-, Myristoyl-, Palmitoyl-, Stearoyl-, Oleoyl- oder Linoyl-Reste umfassen.

**13.** Liposomen umfassend amphotere Lipide nach einem der Ansprüche 1 bis 12, wobei die amphoteren Lipide alternativ zu den Amphiphilen nach Anspruch 1
ein Amphiphil nach der allgemeinen Formel (III) aufweisen können,

(III)

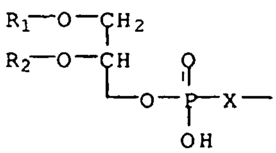

wobei
**R1** und **R2** unabhängig voneinander C8 bis C30 Acylketten mit 0, 1 oder 2 ethylenisch ungesättigten Bindungen sind und **X** -O- ist.

**14.** Liposomen nach Anspruch 13 ein amphoteres Lipid mit der nachfolgender Struktur umfassend

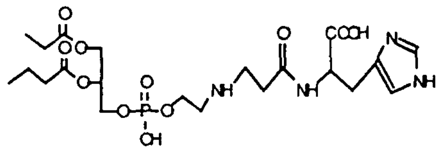

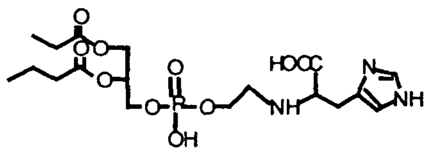

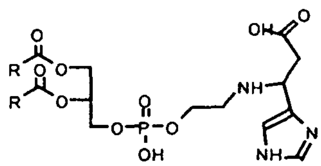

oder

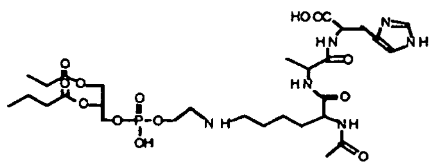

wobei die langkettigen Acyle unabhängig von-einander Lauroyl-, Myristoyl-, Palmitoyl-, Stearoyl-, Oleoyl- und Linoyl-Reste umfassen.

**15.** Liposomen nach Anspruch 13 oder 14,
**dadurch gekennzeichnet, dass**
die Liposomen maximal 50 mol% des amphoteren Lipids umfassen, bevorzugt 2 bis 50 mol%, besonders bevorzugt 10 bis 40 mol%.

**16.** Liposomen nach einem der Ansprüche 13 bis 15,
**dadurch gekennzeichnet, dass**
die Liposomen Phosphatidylcholin, Phosphatidylethanolamin, Diacylglycerol, Tetraetherlipid und/oder PEG-Lipid umfassen.

**17.** Liposomen nach einem der Ansprüche 13 bis 16,
**dadurch gekennzeichnet**, das
die Liposomen eine mittlere Größe zwischen 50 und 1000 nm, bevorzugt zwischen 50 und 300 nm, besonders bevorzugt zwischen 60 und 130 nm aufweisen.

**18.** Liposomen nach einem der Ansprüche 13 bis 17,
**dadurch gekennzeichnet, dass**
die Liposomen einen Wirkstoff umfassen.

**19.** Liposomen nach einem der Ansprüche 13 bis 18,
**dadurch gekennzeichnet, dass**
der Wirkstoff ein Protein, ein Peptid, eine DNA, eine RNA, ein antisense-Nukleotid und/oder ein Decoy-Nukleotid ist.

**20.** Liposomen nach dem Anspruch 18 oder 19,
**dadurch gekennzeichnet, dass**
mindestens 50 $\mu$g, bevorzugt mehr als 90$\mu$g, besonders bevorzugt mehr als 150$\mu$g des Wirkstoffes pro mg Lipid

im Innern des Liposoms eingeschlossen sind.

**21.** Verfahren zur Wirkstoffbeladung von Liposomen nach einem der Ansprüche 13 bis 20,
**dadurch gekennzeichnet, dass**
ein Bindungs-pH-Wert zur Verkapselung eines Wirkstoffs eingesetzt wird und ein zweiter pH-Wert zur Abtrennung der nicht gebundenen Wirkstoffe verwendet wird.

**22.** Liposomen, hergestellt nach dem Verfahren von Anspruch 21.

**23.** Verfahren zur Wirkstoffbeladung von Liposomen nach einem der Ansprüche 13 bis 20,
**dadurch gekennzeichnet, dass**
die Liposomen bei einem bestimmten pH-Wert permeabilisiert und folgend verschlossen werden.

**24.** Verwendung der Liposomen nach einem der Ansprüche 13 bis 20 zur Herstellung von Nanokapseln.

**25.** Verwendung der Liposomen nach einem der Ansprüche 13 bis 20 zur Herstellung von Freisetzungssystemen in der Diagnostik.

**26.** Verwendung der Liposomen nach einem der Ansprüche 13 bis 20 zur Herstellung eines Arzneimittels als Depotformulierung und/oder als zirkulierbares Depot.

**27.** Verwendung der Liposomen nach einem der Ansprüche 13 bis 20 zur Herstellung eines Arzneimittels als Vektor zur Transfektion von Zellen in vivo, in vitro und/oder ex vivo.

**28.** In-vivo-Transfektionssystem,
**dadurch gekennzeichnet, dass**
es mit genetischem Material beladene Liposomen nach Anspruch 13 bis 20 enthält.

**29.** In-vivo-Transfektionssystem nach Anspruch 28,
**dadurch gekennzeichnet, dass**
das Amphiphil eine Struktur nach der Formel (V)

(V)

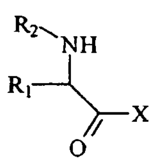

wobei
**R1** und **R2** unabhängig voneinander C8 bis C30 Alkyl mit 0, 1 oder 2 ethylenisch ungesättigten Bindungen sind und
**X** ausgewählt ist aus der Gruppe -O-; -NH-; -S-
oder
nach der der Formel (VI):

(VI)

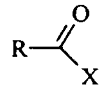

wobei
**R** C8 bis C30 Alkyl mit 0, 1 oder 2 ethylenisch ungesättigten Bindungen ist und
**X** ausgewählt ist aus der Gruppe -O-; -NH-; -S-

aufweisen kann.

**30.** Verwendung des In-vivo-Transfektionssystems nach Anspruch 28 oder 29 zur Herstellung eines Arzneimittels als Vektor zur Transfektion von Zellen in vivo, in vitro und/oder ex vivo.

**31.** Verwendung der Liposomen nach einem der Ansprüche 13 bis 20 zur Herstellung eines Arzneimittels bei intravenöser und/oder peritonealer Applikation.

## Claims

**1.** An amphoteric lipid, which lipid has an isoelectric point between 4.5 and 8.5, according to general formula (I):

(I) Amphoteric substance - Y - spacer - amphiphilic substance

**characterized in that**

(a) the amphoteric substance has at least one portion of cationic charge with a pKa value between 4 and 8 and/or at least one portion of anionic charge with a pKa value between 3 and 7 and optionally additional charge carriers,

aa) said portion of cationic charge being selected from the group comprising imidazole, morpholine, piperazine, purine, pyridine and/or pyrimidine or derivatives thereof,
bb) said portion of anionic charge being a carboxyl group which comprises acetic acid, bromoacetic acid, chloroacetic acid, acetoacetic acid, propionic acid, acrylic acid, butyric acid, crotonic acid, or carboxylic acids bound in the aliphatic chain; which comprises dicarboxylic acids that are mono-esterified or amidated or bound in the aliphatic chain; which comprises oligocarboxylic acids that are mono-esterified or amidated or bound in the aliphatic chain,

(b) the spacer is a lower alkyl residue with up to 8 C atoms, with linear, branched or cyclic structure, with 0, 1 or 2 ethylenically unsaturated bonds, and 0-4 hydroxyl groups,
(c) Y is absent or comprises -(C=O)-O-; -(C=O)-NH-; -NH-(C=O)-O-; -O-; -NH-; -CH=N-; -O-(O=C)-; -S-; -(O=C)-; -NH-(O=C)-; -O-(O=C)-NH-, -N=CH- and/or -S-S-,
(d) the amphiphilic substance is a structure according to general formula (II) or (IV):

(II)

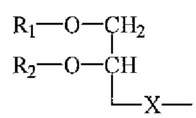

wherein
$R_1$ and $R_2$ independently are $C_8$-$C_{30}$ alkyl or acyl chains with 0, 1 or 2 ethylenically unsaturated bonds, and
X is absent or selected from the group comprising -O-(C=O); -NH-(C=O)-; -S-(C=O)-; -O-; -NH-; -S-; -N=CH-; -(O=C)-O-; -S-(O=C)-; -NH-(O=C)-; -N=CH- and/or -S-S-;
or

(IV)

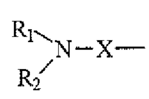

wherein
$R_1$ and $R_2$ independently are $C_8$-$C_{30}$ alkyl chains with 0, 1 or 2 ethylenically unsaturated bonds, and
X is absent or selected from the group consisting of -(C=O)-O-; -(C=O)-NH-; -(C=O)-S-; -NH-; -CH=N-; and/or -S-(O=C)-;

(e) the amphiphilic substance is a 1,4- or 1,5-dicarboxylic acid such as aspartic acid, glutamic acid, malic acid, tartaric acid, citric acid, aconitic acid, citraconic acid and/or maleic acid esterified with linear $C_8$-$C_{30}$ alcohols, and/or

(f) the amphiphilic substance is a 1,4- or 1,5-diamine of 3-aminoalanine, diaminobutyric acid, ornithine, or lysine amidated with linear $C_8$-$C_{30}$ fatty acids.

**2.** The lid according to claim 1,
**characterized in that**
the dicarboxylic acid is selected from the group comprising oxalic acid, malonic acid, succinic acid, maleic acid, fumaric acid, malic acid, tartaric acid, glutaric acid, adipic acid, caprylic acid, pimelic acid, suberic acid, cyclohexanedicarboxylic acid and/or cyclopentanedicarboxylic acid, and/or the oligocarboxylic acid is selected from the group comprising citric acid, isocitric acid and/or ethylenediaminetetraacetic acid.

**3.** The lipid according to claim 1 or 2,
**characterized in that**
the amphoteric lipid has an isoelectric point between 5 and 7.

**4.** The lipid according to any of claims 1 to 3,
**characterized in that**
the pKa values of the portions of cationic and anionic charge in the amphoteric substance are 2 pH units apart at maximum.

**5.** The lipid according to any of claims 1 to 4,
**characterized in that**
the portion of cationic charge of the amphoteric substance comprises imidazole, piperazine, morpholine and the portion of anionic charge comprises the carboxyl group.

**6.** The lipid according to any of claims 1 to 5,
**characterized in that**
the amphoteric substance is a peptide with 2-6 amino acids selected from the group consisting of histidine, arginine, lysine, glutamic acid and/or aspartic acid,
wherein

i) the percentage of His and Asp/Glu does not exceed 66%,
ii) the percentage of Arg/Lys is smaller than 50% and that of Asp/Glu higher than 50%, or
iii) the percentage of His and Arg/Lys is smaller than/equal to 33% and that of Asp/Glu is higher than that of Arg/Lys.

**7.** The lipid according to any of claims 1 to 6,
**characterized in that**
the amphiphilic substance is selected from the group consisting of diacylglycerols, dialkylglycerols, phosphoglycerols, acylated or alkylated 3-amino-1,2-propanediols and/or N,N-dialkylamines.

**8.** The lipid according to any of claims 1 to 7,
**characterized in that**
the spacer is a sugar or a polyethylene glycol with up to 20 monomer units.

**9.** An amphoteric lipid according to one of the preceding claims, which lipid has the structure

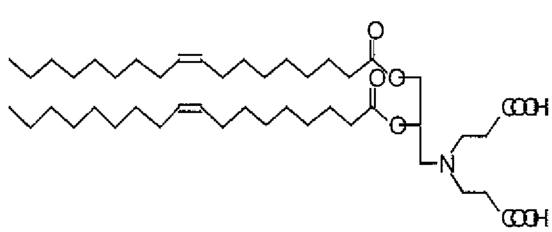

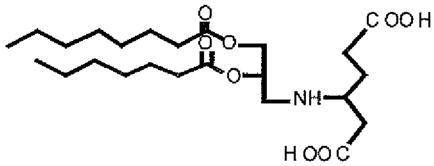

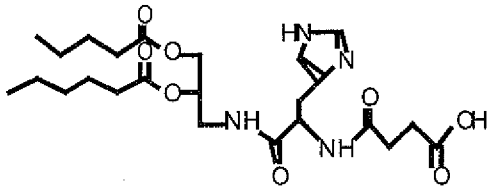

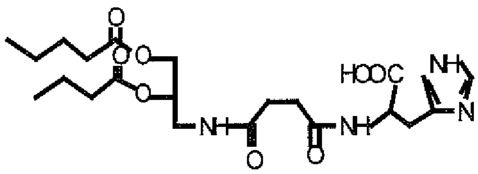

or

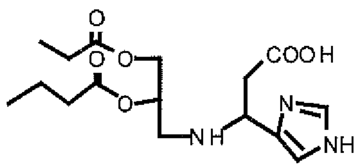

wherein the long-chain alkyl or acyl residues independently comprise

a) lauroyl, myristoyl, palmitoyl, stearoyl, oleoyl or linoleoyl residues, or
b) lauryl, myristyl, palmityl, stearyl, oleyl or linoleyl residues.

**10.** An amphoteric lipid according to one of the preceding claims, which lipid has the structure

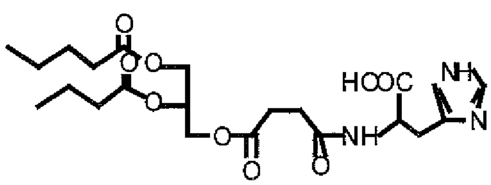

or

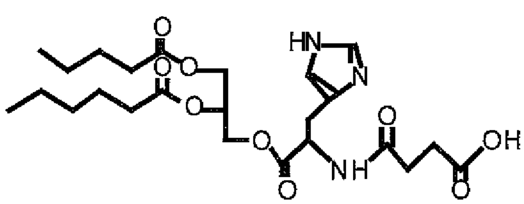

wherein the long-chain alkyl or acyl residues independently comprise

a) lauroyl, myristoyl, palmitoyl, stearoyl, oleoyl or linoleoyl residues, or
b) lauryl, myristyl, palmityl, stearyl, oleyl or linoleyl residues.

**11.** An amphoteric lipid according to one of the preceding claims, which lipid has the structure

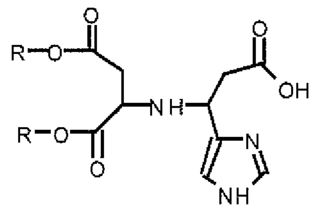

or

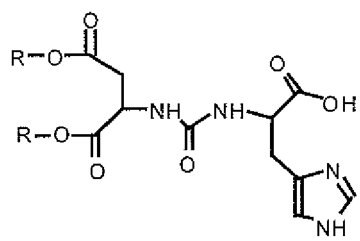

wherein the long-chain alkyl residues independently comprise lauryl, myristyl, palmityl, stearyl, oleyl or linoleyl residues.

**12.** An amphoteric lipid according to one of the preceding claims, which lipid has the structure

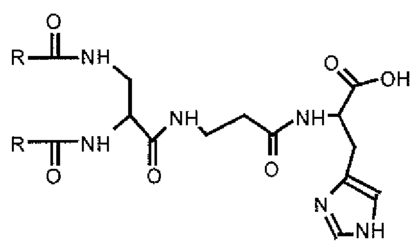

wherein the long-chain acyl residues comprise lauroyl, myristoyl, palmitoyl, stearoyl, oleoyl or linoleoyl residues.

**13.** Liposomes comprising the amphoteric lipids according to any of claims 1 to 12, wherein the the amphoteric lipids comprise alternatively to the amphiphilic substances according to claim 1 an amphiphilic substance according to general formula (III):

(III)

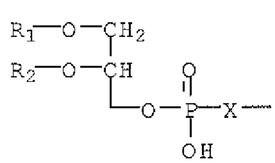

wherein
$R_1$ and $R_2$ independently are $C_8$-$C_{30}$ acyl chains with 0, 1 or 2 ethylenically unsaturated bonds, and
X is -O-.

**14.** The liposomes according to claim 13 comprising an amphoteric lipid, which lipid has the structure

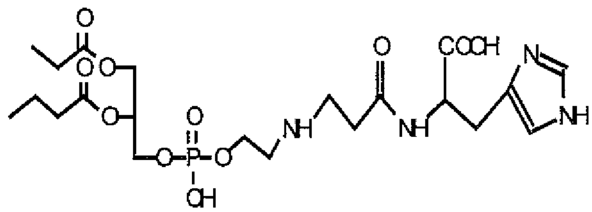

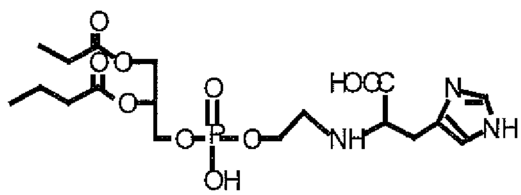

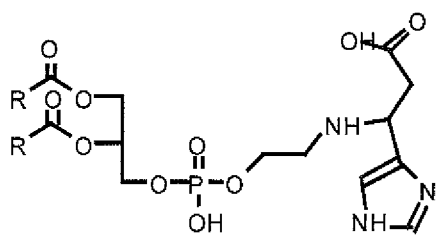

or

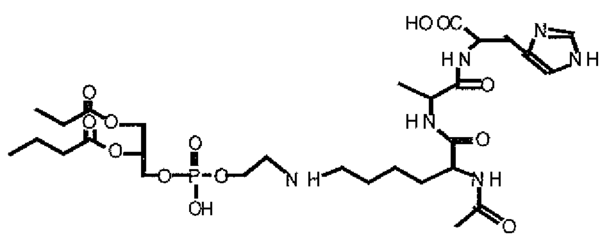

wherein the long-chain acyl residues independently comprise lauroyl, myristoyl, palmitoyl, stearoyl, oleoyl and linoleoyl residues.

**15.** The liposomes according to claims 13 to 14,
**characterized in that**
the liposomes comprise 50 mole-% of amphoteric lipid at maximum, preferably 2 to 50 mole-%, more preferably 10 to 40 mole-%.

**16.** The liposomes according to claim 13 to 15,
**characterized in that**
the liposomes comprise phosphatidyl choline, phosphatidyl ethanolamine, diacylglycerol, tetraether lipid and/or PEG lipid.

**17.** The liposomes according to any of claims 13 to 16,
**characterized in that**
the liposomes have an average size between 50 and 1000 nm, preferably between 50 and 300 nm, and more preferably between 60 and 130 nm.

**18.** The liposomes according to any of claims 13 to 17,
**characterized in that**
the liposomes comprise an active substance.

**19.** The liposomes according to any of claims 13 to 18,
**characterized in that**
the active substance is a protein, a peptide, a DNA, an RNA, an antisense nucleotide and/or a decoy nucleotide.

**20.** The liposomes according to claim 18 or 19,
**characterized in that**
at least 50 μg, preferably more than 90 μg, and more preferably more than 150 μg of active substance per mg lipid is entrapped inside the liposome.

**21.** A method of loading the liposomes according to any of claims 13 to 20 with active substance,
**characterized in that**
a binding pH value for encapsulation of an active substance and a second pH value for removal of unbound active substance is used.

**22.** Liposomes, produced according to the method of claim 21.

**23.** A method of loading the liposomes according to any of claims 13 to 20 with active substance,
**characterized in that**
the liposomes are made permeable at a specific pH value and sealed thereafter.

**24.** Use of the liposomes according to any of claims 13 to 20 in the production of nanocapsules.

**25.** Use of the liposomes according to any of claims 13 to 20 in the production of release systems for use in diagnostics.

**26.** Use of the liposomes according to any of claims 13 to 20 for the production of a pharmaceutical composition for use as a depot formulation and/or as a circulative depot.

**27.** Use of the liposomes according to any of claims 13 to 20 for the production of a pharmaceutical composition for use as a vector to transfect cells *in vivo, in vitro* and/or *ex vivo.*

**28.** An *in vivo* transfection system,
**characterized in that**
said system includes the liposomes according to any of claims 13 to 20 loaded with genetic material.

**29.** The *in vivo* transfection system according to claim 28,
**characterized in that**
the amphiphilic substance is a structure according to general formula (V)

(V)

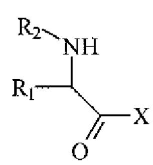

wherein
$R_1$ and $R_2$ independently are $C_8$-$C_{30}$ alkyl with 0, 1 or 2 ethylenically unsaturated bonds, and
X is selected from the group of -0-; -NH-; -S-

or
the amphiphilic substance is a structure according to formula (VI):

(VI)

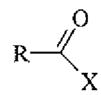

wherein
R is $C_8$-$C_{30}$ alkyl with 0, 1 or 2 ethylenically unsaturated bonds, and
X is selected from the group of -O-; -NH-; -S-.

**30.** Use of the *in vivo* transfection system according to claim 28 or 29 as a vector to transfect cells *in vivo, in vitro* and/or *ex vivo.*

**31.** Use of the liposomes according to any of claims 13 to 20 for the production of pharmaceutical composition for use in intravenous and/or peritoneal applications.

**Revendications**

**1.** Lipide amphotère ayant un point isoélectrique entre 4,5 et 8,5 selon la formule générale (I) :

(I) amphotère - Y - espaceur - amphiphile

**caractérisé en ce que**

(a) l'amphotère comprend au moins une partie de charge cationique ayant un pKa entre 4 et 8 et/ou au moins une partie de charge anionique ayant un pKa entre 3 et 7 et éventuellement d'autres porteurs de charge, moyennant quoi

aa) la partie de charge cationique est choisie dans le groupe comprenant l'imidazole, la morpholine, la pipérazine, la purine, la pyridine et/ou le pyrimidine ou un dérivé desquelles,
bb) la partie de charge anionique est un groupe carboxyle, qui comprend de l'acide acétique, de l'acide bromoacétique, de l'acide chloroacétique, de l'acide acétylacétique, de l'acide propionique, de l'acide acrylique, de l'acide butyrique, de l'acide crotonique ou des acides carboxyliques liés dans la chaîne aliphatique, qui comprend des acides dicarboxyliques mono estérifiés ou amidifiés ou liés dans la chaîne aliphatique, qui comprend des acides oligocarboxyliques mono estérifiés ou amidifiés ou liés dans la chaîne aliphatique,

(b) l'espaceur est un radical alkyle inférieur comprenant 0 à 8 atomes de carbone et présentant une structure linéaire, ramifiée ou cyclique comportant 0, 1 ou 2 liaisons éthylèniquement insaturées et 0 à 4 groupes hydroxyle,
(c) Y est absent ou comprend -(C=O)-O-, -(C=O)-NH-, -NH-(C=O)-O-, -O-, -NH-, -CH=N-, -O-(O=C)-, -S-, (O=C)-, -NH-(O=C)-, -O-(O=C)-NH-, -N=CH- et/ou -S-S-,
(d) l'amphiphile est une structure selon la formule générale (II) ou (IV) :

(II)

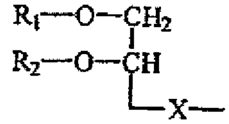

dans laquelle
R1 et R2 sont indépendamment l'un de l'autre des chaînes alkyle ou acyle $C_8$-$C_{30}$ comportant 0, 1 ou 2 liaisons

éthylèniquement insaturées et
X est absent ou choisi dans le groupe comprenant -O-(C=O), -NH-(C=O), -S-(C=O)-, -O-, -NH-, -S-, -N=CH-, -(O=C)-O, -S-(O=C)-, -NH-(O=C)-, -N=CH- et/ou -S-S-,
ou

(IV)

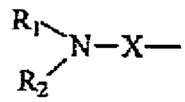

dans laquelle
R1 et R2 sont indépendamment l'un de l'autre des chaînes alkyle en $C_8$-$C_{30}$ comportant 0, 1 ou 2 liaisons éthylèniquement insaturées et X est absent ou choisi dans le groupe constitué de -(C=O)-O-, -(C=O)-NH-, -(C=O)-S-, -NH-, -CH=N- et/ou -S-(O=C)-,
(e) l'amphiphile est un acide 1,4- ou 1,5-dicarboxylique tel que l'acide aspartique, l'acide glutamique, l'acide malique, l'acide tartrique, l'acide citrique, l'acide aconitique, l'acide citraconique et/ou l'acide maléïque estérifié par des alcools linéaires en $C_8$-$C_{30}$ et/ou
(f) l'amphiphile est un 1,4- ou 1,5-diamine de 3-aminoalanine, acide diaminobutyrique, ornithine ou lysine amidifié avec des acides gras linéaires en $C_8$-$C_{30}$.

**2.** Lipide selon la revendication 1, **caractérisé en ce que**
l'acide dicarboxylique est choisi dans le groupe comprenant l'acide oxalique, l'acide malonique, l'acide succinique, l'acide maléïque, l'acide fumarique, l'acide malique, l'acide tartrique, l'acide glutarique, l'acide adipique, l'acide caprylique, l'acide pimélique, l'acide subérique, l'acide cyclohexane-dicarboxylique, et/ou l'acide cyclopentane-dicarboxylique et/ou l'acide oligocarboxylique est choisi dans le groupe comprenant l'acide citrique, l'acide isocitrique et/ou l'acide éthylènediamine-tétraacétique.

**3.** Lipide selon la revendication 1 ou 2,
**caractérisé en ce que**
le lipide amphotère a un point isoélectrique entre 5 et 7.

**4.** Lipide selon l'une des revendications 1 à 3,
**caractérisé en ce que**
les valeurs de pKa des charges partielles cationique et anionique de l'amphotère se différencient au maximum de 2 unités de pH.

**5.** Lipide selon l'une des revendications 1 à 4,
**caractérisé en ce que**
la partie de charge cationique de l'amphotère comprend de l'imidazole, de la pipérazine, de la morpholine, et la partie de charge anionique comprend le groupe carboxyle.

**6.** Lipide selon l'une des revendications précédentes 1 à 5,
**caractérisé en ce que**
l'amphotère est un peptide comportant 2 à 6 acides aminés, qui sont choisis dans le groupe constitué de l'histidine, l'arginine, la lysine, l'acide glutamique et/ou l'acide aspartique,
moyennant quoi

i) la proportion en pourcentage de His et Asp/Glu n'est pas supérieure à 66 % ou
ii) la proportion en pourcentage de Arg/Lys est inférieure à 50 %, la proportion d'Asp/Glu est supérieure à 50 %, ou
iii) la proportion en pourcentage de His et Arg/Lys est inférieure ou égale à 33 %, celle d'Asp/Glu est supérieure à celle d'Arg/Lys.

**7.** Lipide selon l'une des revendications précédentes 1 à 6,
**caractérisé en ce que**
l'amphiphile est sélectionné dans le groupe constitué des diacylglycérols, des dialkylglycérols, des phosphoglycérols, des 3-amino-1,2-propanediols et/ou des N,N-dialkylamines acylés ou alkylés.

8. Lipide selon l'une des revendications précédentes 1 à 7,
**caractérisé en ce que**
l'espaceur est du sucre ou un polyéthylèneglycol ayant jusqu'à 20 unités monomères.

9. Lipide amphotère selon l'une des revendications précédentes ayant la structure :

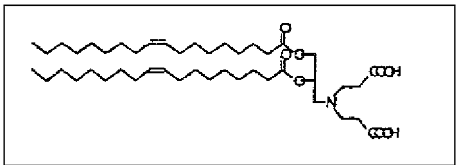

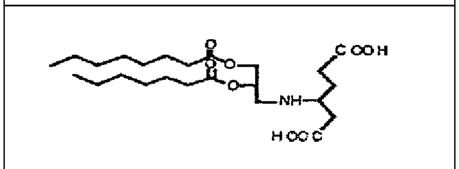

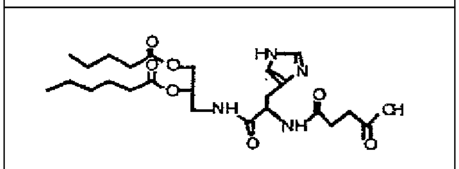

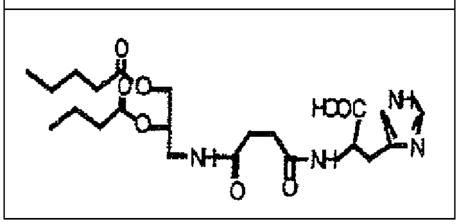

ou

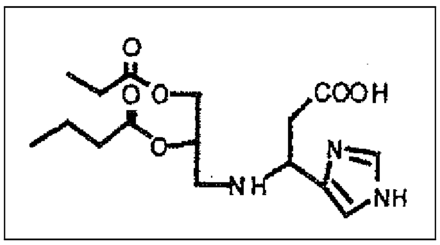

dans lesquelles les alkyles ou acyles à longue chaîne comprennent indépendamment l'un de l'autre

a) des radicaux lauroyl, myristoyl, palmitoyl, stéaroyl, oléoyl ou linoyl ou
b) des radicaux lauryl, myristyl, palmityl, stéaryl, oléyl, ou linyl.

10. Lipide amphotère selon l'une des revendications précédentes ayant la structure

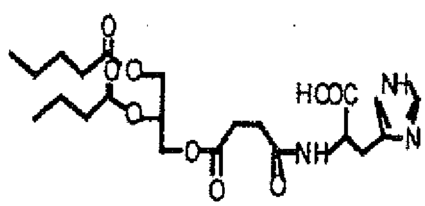

ou

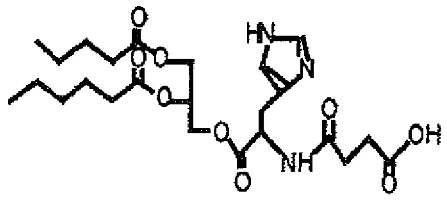

dans lesquelles les alkyles ou acyles à longue chaîne comprennent indépendamment l'un de l'autre

a) des radicaux lauroyl, myristoyl, palmitoyl, stéaroyl, oléoyl ou linoyl ou
b) des radicaux lauryl, myristyl, palmityl, stéaryl, oléyl, ou linyl.

**11.** Lipide amphotère selon l'une des revendications précédentes ayant la structure

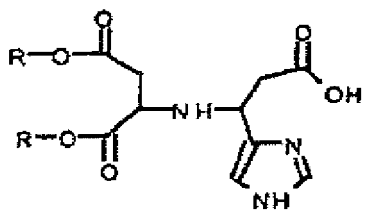

ou

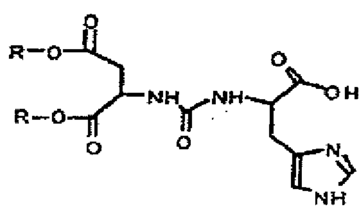

dans lesquelles les alkyles à longue chaîne comprennent indépendamment l'un de l'autre des radicaux lauryl, myristyl, palmityl, stéaryl, oléyl, ou linyl.

**12.** Lipide amphotère selon l'une des revendications précédentes ayant la structure :

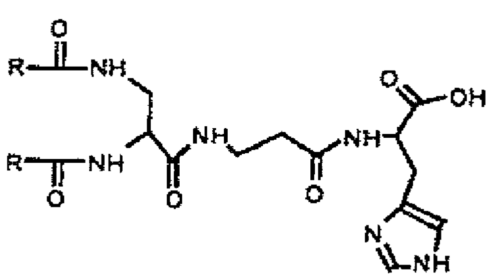

dans laquelle les acyles à longue chaîne comprennent indépendamment l'un de l'autre des radicaux lauroyl, myristoyl, palmitoyl, stéaroyl, oléoyl ou linoyl.

**13.** Liposomes comprenant des lipides amphotères selon l'une des revendications 1 à 12, dans lesquels les lipides amphotères peuvent comporter, de manière alternative aux amphiphiles selon la revendication 1,
un amphiphile selon la formule générale (III)

(III)

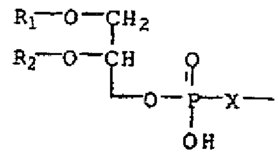

dans laquelle
R1 et R2 sont indépendamment l'un de l'autre des chaînes acyle en $C_8$-$C_{30}$ comportant 0, 1 ou 2 liaisons éthyléniquement insaturées et
X est -O-.

**14.** Liposomes selon la revendication 13 comprenant un lipide amphotère ayant la structure suivante

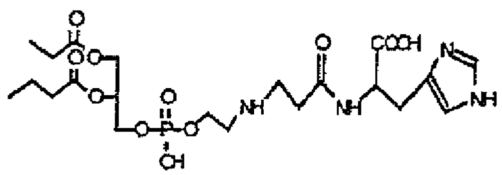

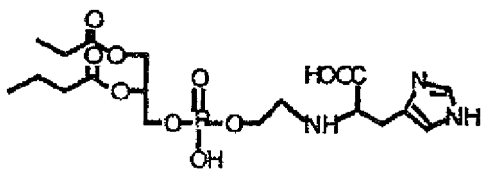

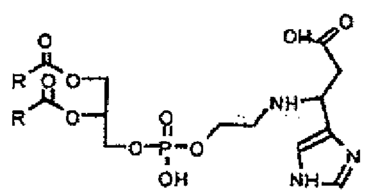

ou

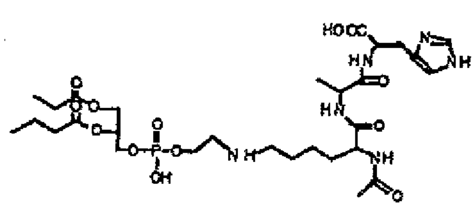

dans lesquelles les acyles à longue chaîne comprennent indépendamment l'un de l'autre des radicaux lauroyl, myristoyl, palmitoyl, stéaroyl, oléoyl ou linoyl.

**15.** Liposomes selon la revendication 13 ou 14,
**caractérisés en ce que**
les liposomes comprennent au maximum 50 % en moles du lipide amphotère, de préférence de 2 à 50 % en moles

et de manière plus préférée de 10 à 40 % en moles.

16. Liposomes selon l'une des revendications 13 à 15, **caractérisés en ce que** les liposomes comprennent de la phosphatidylcholine, du phosphatidyléthanolamine, du diacylglycérol, un tétraétherlipide ou un lipide PEG.

17. Liposomes selon l'une des revendications 13 à 16, **caractérisés en ce que** les liposomes ont une grandeur moyenne comprise entre 50 et 1000 nm, de préférence entre 50 et 300 nm et de manière encore plus préférée entre 60 et 130 nm.

18. Liposomes selon l'une des revendications 13 à 17, **caractérisés en ce que** les liposomes contiennent un principe actif.

19. Liposomes selon l'une des revendications 13 à 18, **caractérisés en ce que** le principe actif est une protéine, un lipide, un ADN, un ARN, un nucléotide antisens et/ou un nucléotide decoy.

20. Liposomes selon la revendication 18 ou 19, **caractérisés en ce que** au moins 50 μg, de préférence plus de 90 μg, de manière encore plus préférée plus de 150 μg du principe actif par mg de lipide sont inclus à l'intérieur du liposome.

21. Procédé de charge de principe actif dans les liposomes selon l'une des revendications 13 à 20, **caractérisé en ce qu'**on applique un pH de liaison pour encapsuler le principe actif et qu'on applique un second pH pour séparer le principe actif non lié.

22. Liposomes, préparés selon le procédé de la revendication 21.

23. Procédé de charge de principe actif dans les liposomes selon l'une des revendications 13 à 20, **caractérisé en ce qu'**on rend perméable les liposomes à un certain pH et qu'on les obture ensuite.

24. Utilisation des liposomes selon l'une des revendications 13 à 20 pour préparer des nanocapsules.

25. Utilisation des liposomes selon l'une des revendications 13 à 20 pour préparer des systèmes de libération pour le diagnostic.

26. Utilisation des liposomes selon l'une des revendications 13 à 20 pour préparer un médicament sous forme de formulation de retard ou de forme retard pouvant circuler.

27. Utilisation des liposomes selon l'une des revendications 13 à 20 pour préparer un médicament servant de vecteur de transfection de cellules in vivo, in vitro et/ou ex vivo.

28. Système de transfection in vivo, **caractérisé en ce qu'**il contient des liposomes selon la revendication 13 à 20 chargés avec un matériau génétique.

29. Système de transfection in vivo selon la revendication 28, **caractérisé en ce que** l'amphiphile peut avoir une structure selon la formule (V)

(V)

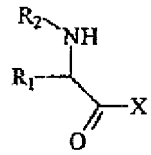

dans laquelle
R1 et R2 sont indépendamment l'un de l'autre un $C_8$-$C_{30}$ alkyle comportant 0, 1 ou 2 liaisons éthylèniquement insaturées et
X est choisi dans le groupe comprenant -O-, -NH-, -S-,
ou
selon la formule (VI) :

(VI)

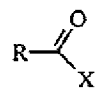

dans laquelle
R est un alkyle en $C_8$-$C_{30}$ comportant 0, 1 ou 2 liaisons éthylèniquement insaturées et
X est choisi dans le groupe comprenant -O-, -NH-, -S-.

**30.** Utilisation du système de transfection in vivo selon la revendication 28 ou 29 pour préparer un médicament servant de vecteur de transfection de cellules in vivo, in vitro et/ou ex vivo.

**31.** Utilisation des liposomes selon l'une des revendications 13 à 20 pour préparer un médicament destiné à une administration par voie intraveineuse et/ou péritonéale.

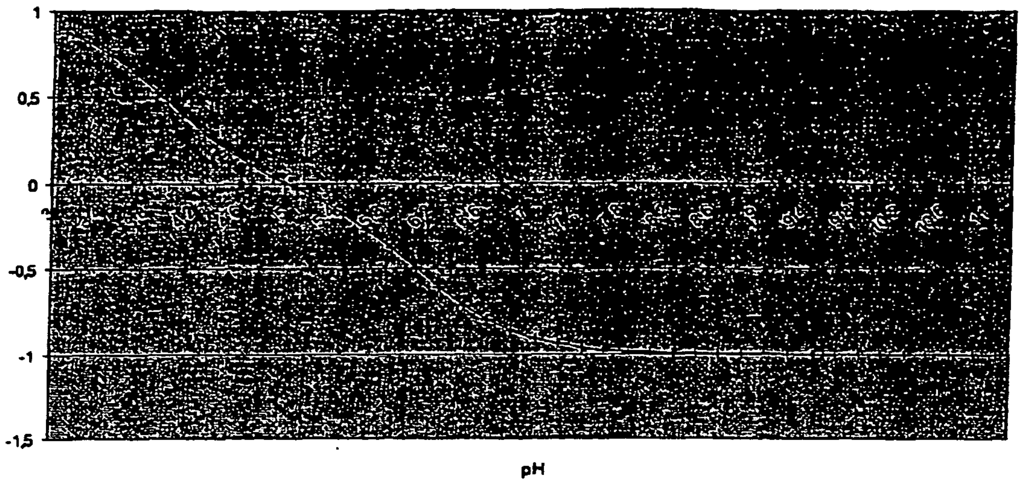


Anmeldungsgemäße Verbindungen #34, #35 1x His pK 6, 1x Asp pK 3,86

Abb. 1

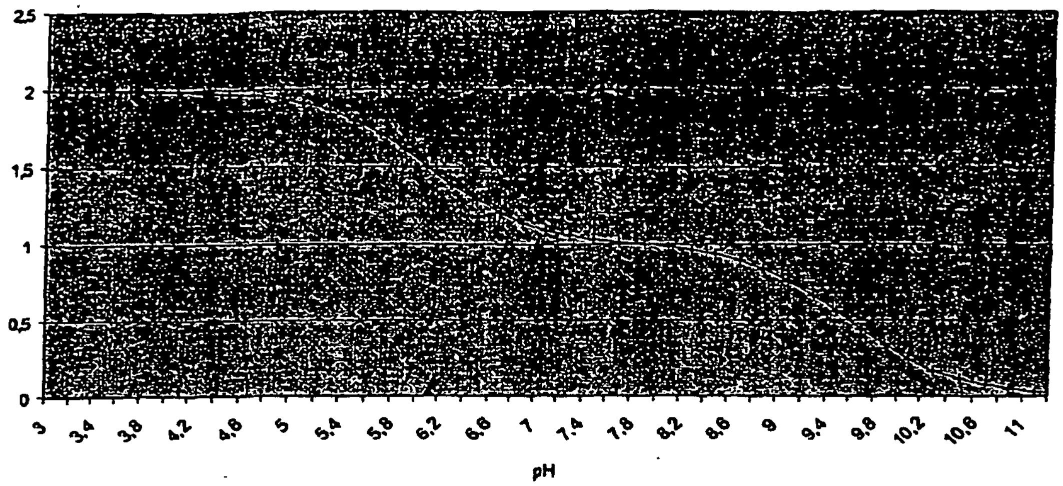


Verbindung 54, 55 nach D1: 1x His pK 6 + 1x NH2 pK 9,5

**Abb. 2**

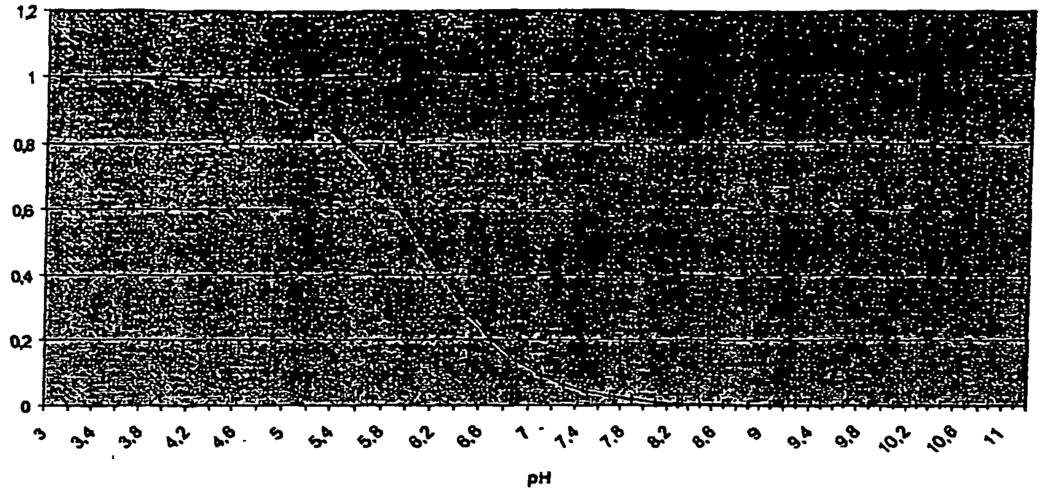


Verbindung aus D3 R1: Acetyl, R2: His, pK 6

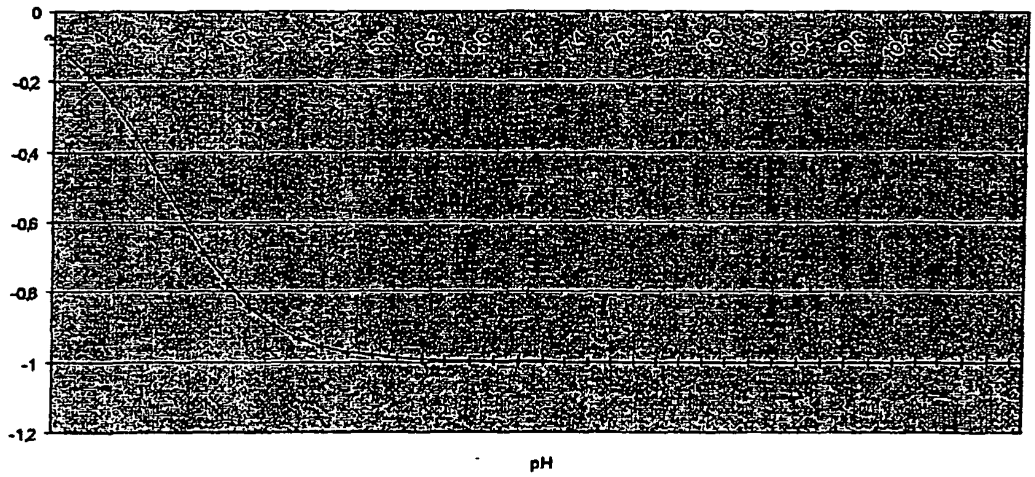


Verbindung aus D3 R1: Acetyl, R2: Asp, pK 3,86

Abb. 3